# EUROPÄISCHE PATENTSCHRIFT

(11) **EP 2 817 297 B1**
(45) Veröffentlichungstag und Bekanntmachung des Hinweises auf die Patenterteilung: **17.02.2016**
(21) Anmeldenummer: 13704472.3
(22) Anmeldetag: 18.02.2013
(51) Int. Cl.: C07D 249/14, C07D 257/06, C07D 271/08, C07D 271/113, C07D 409/12, C07D 411/12, C07D 413/12, A01N 43/707, A01N 43/713, A01N 43/824, A01N 43/832

(54) **HERBIZID WIRKSAME SULFINYLAMINOBENZAMIDE**
HERBICIDALLY ACTIVE 3-(SULFINYLAMINO)-BENZAMIDES
3-(SULFINYLAMINO)-BENZAMIDES À ACTIVITÉ HERBICIDE

(30) Priorität: 21.02.2012 EP 12156307
(43) Veröffentlichungstag der Anmeldung: 31.12.2014
(73) Patentinhaber: Bayer Intellectual Property GmbH, 40789 Monheim am Rhein (DE)
(72) Erfinder: AHRENS, Hartmut, 63329 Egelsbach (DE); BRAUN, Ralf, 76857 Ramberg (DE); KÖHN, Arnim, 55270 Klein-Winternheim (DE); LEHR, Stefan, 69006 Lyon (FR); DIETRICH, Hansjörg, 65835 Liederbach am Taunus (DE); SCHMUTZLER, Dirk, 65795 Hattersheim (DE); GATZWEILER, Elmar, 61231 Bad Nauheim (DE); ROSINGER, Christopher, Hugh, 65719 Hofheim (DE)
(74) Vertreter: BIP Patents
(86) Internationale Anmeldenummer: PCT/EP2013/053176
(87) Internationale Veröffentlichungsnummer: WO 2013/124238

(56) Entgegenhaltungen:
- WO-A1-2004/052849
- WO-A1-2011/035874
- US-A1- 2011 144 345

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 2011/035874 A1 sind herbizid wirksame N-(1,2,5-Oxadiazol-3-yl)benzamide bekannt. WO 2004/052849 A1 offenbart herbizid wirksame Benzoyl-Derivate, die in 3-Position des Phenylrings eine Sulfinylamino-Gruppe tragen. Die herbizide Wirksamkeit und/oder die Kulturpflanzenverträglichkeit der in diesen Schriften genannten Verbindungen ist jedoch nicht immer ausreichend.

Aufgabe der vorliegenden Erfindung war die Bereitstellung von herbizid wirksamen Verbindungen mit gegenüber den aus dem Stand der Technik bekannten Verbindungen verbesserten Eigenschaften.

Es wurde nun gefunden, dass bestimmte Sulfinylaminobenzamide als Herbizide besonders gut geeignet sind.

Ein Gegenstand der vorliegenden Erfindung sind somit Sulfinylaminobenzamide der Formel (I) oder deren Salze 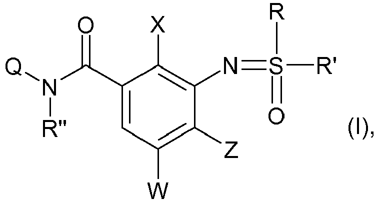 worin die Symbole und Indizes folgende Bedeutungen haben:
Q bedeutet einen Rest Q1, Q2, Q3 oder Q4, 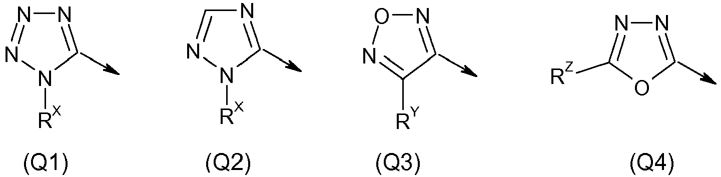
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Z bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C,
(R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
W bedeutet Wasserstoff, Halogen, Nitro, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, Halogen-(C₃-C₇)-cycloalkyl, (C₁-C₆)-Alkoxy, Halogen-(C₁-C₆)-alkoxy, (C₁-C₆)-Alkyl-(O)ₙS-, (C₁-C₆)-Halogenalkyl-(O)ₙS-, (C₁-C₆)-Alkoxy-(C₁-C₄)-alkyl, (C₁-C₆)-Alkoxy-(C₁-C₄)-halogenalkyl, R¹(O)C, R¹(R¹ON=)C, R¹O(O)C, (R¹)₂N, R¹(O)C(R¹)N oder R²(O)₂S(R¹)N,
R und R' bedeuten unabhängig voneinander jeweils (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
oder R und R' bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außer dem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R" bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C, R¹O(O)C, (R¹)₂N(O)C, R¹O, (R¹)₂N, R²O(O)C(R¹)N, (R¹)₂N(O)C(R¹)N, R²(O)₂S, oder jeweils durch s Reste aus der Gruppe bestehend aus Methyl, Ethyl, Methoxy, Nitro, Trifluormethyl und Halogen substituiertes Benzyl,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus Nitro, Cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, und wobei die vier letztgenannten Reste durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfanyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl, und wobei Heterocyclyl n Oxogruppen trägt,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, R¹O, R¹(H)N, Methoxycarbonyl, Ethoxycarbonyl, Methylcarbonyl, Dimethylamino, Trifluormethylcarbonyl, Acetylamino, Methylsulfanyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl,
R⁷ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
n bedeutet 0, 1 oder 2,
m bedeutet 0, 1, 2, 3 oder 4,
s bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn in der Gruppierung S(O)ₙ n für 1 steht. Stereoisomere treten ebenfalls auf, wenn die Reste R und R' unterschiedliche Bedeutungen haben. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle von R". Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR*R**R**]⁺, worin R, R*, R** und R*** unabhängig voneinander jeweils einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Bevorzugt sind Verbindungen der allgemeinen Formel (I), worin
Q bedeutet einen Rest Q1, Q2, Q3 oder Q4, 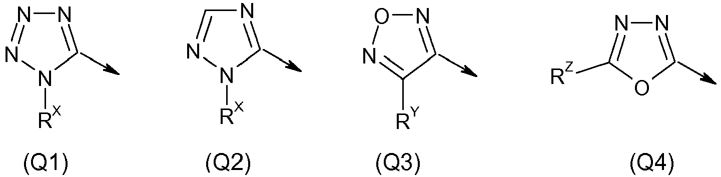
X bedeutet Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Z bedeutet Wasserstoff, Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
W bedeutet Wasserstoff, Halogen, Nitro, Cyano, (C₁-C₆)-Alkyl, Halogen-(C₁-C₆)-alkyl, (C₃-C₇)-Cycloalkyl, (C₁-C₆)-Alkoxy, (C₁-C₆)-Alkyl-(O)ₙS-, R¹O(O)C, (R¹)₂N, R¹(O)C(R¹)N oder R²(O)₂S(R¹)N,
R und R' bedeuten unabhängig voneinander jeweils (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, (C₁-C₆)-Alkyl, Halogen-(C₁-C₆)-alkyl, R¹O(O)C, (R¹)₂N(O)C, R¹O, (R¹)₂N, R²(O)ₙS, und R¹O-(C₁-C₆)-Alkyl substituiert sind, und wobei Heterocyclyl n Oxogruppen trägt,
oder R und R' bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außer dem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Halogen, (C₁-C₆)-Alkyl, Halogen-(C₁-C₆)-alkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R" bedeutet Wasserstoff,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy und Halogen substituiert sind, und wobei Heterocyclyl n Oxogruppen trägt,
oder R^{X} bedeutet (C₃-C₇)-Cycloalkyl, wobei dieser Rest jeweils durch s Reste aus der Gruppe bestehend aus Halogen, (C₁-C₆)-Alkyl und Halogen-(C₁-C₆)-alkyl substituiert ist,
R^{Y} bedeutet Wasserstoff, (C₁-C₆)-Alkyl, Halogen-(C₁-C₆)-alkyl, (C₃-C₇)-Cycloalkyl, (C₁-C₆)-Alkoxy, Methoxycarbonyl, Methoxycarbonylmethyl, Halogen, Amino, Aminocarbonyl oder Methoxymethyl,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, R¹O, R¹(H)N, Methoxycarbonyl, Acetylamino oder Methylsulfonyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R⁵ bedeutet Wasserstoff oder (C₁-C₄)-Alkyl,
R⁷ bedeutet Acetoxy, Acetamido, Methoxycarbonyl oder (C₃-C₆)-Cycloalkyl,
n bedeutet 0, 1 oder 2,
m bedeutet 0, 1 oder 2,
s bedeutet 0, 1, 2 oder 3.

Besonders bevorzugt sind Verbindungen der allgemeinen Formel (I), worin
Q bedeutet einen Rest Q1, Q2, Q3 oder Q4, 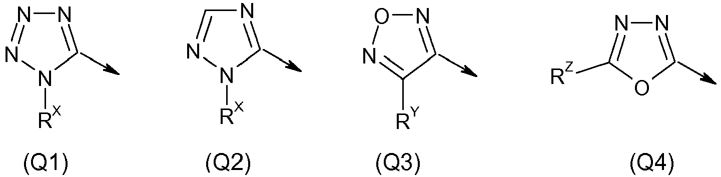
X bedeutet Nitro, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Hydroxycarbonyl, Methoxycarbonyl, Ethoxycarbonyl, Methoxy, Ethoxy, Methylsulfanyl, Methylsulfinyl, Methylsulfonyl, Methoxymethyl, Ethoxymethyl, Methoxyethyl, Methoxyethoxymethyl, Methylthiomethyl, Methylsulfinylmethyl oder Methylsulfonylmethyl,
Z bedeutet Wasserstoff, Nitro, Cyano, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Hydroxycarbonyl, Methoxycarbonyl, Ethoxycarbonyl, Methoxy, Ethoxy, Methylsulfanyl, Methylsulfinyl oder Methylsulfonyl,
W bedeutet Wasserstoff, Chlor oder Methyl,
R und R' bedeuten unabhängig voneinander jeweils Methyl, Ethyl oder n-Propyl, oder
R und R' bilden gemeinsam mit dem Schwefel-Atom, an dem sie gebunden sind, einen 5- oder 6-gliedrigen gesättigten Ring, der außer den Kohlenstoffatomen und außer dem Schwefelatom der Sulfoximinogruppe m Sauerstoffatome enthält,
R" bedeutet Wasserstoff,
R^{X} bedeutet Methyl, Ethyl, n-Propyl, Prop-2-en-1-yl, Methoxyethyl, Ethoxyethyl oder Methoxyethoxyethyl,
R^{Y} bedeutet Methyl, Ethyl, n-Propyl, Chlor oder Amino,
R^{Z} bedeutet Methyl, Ethyl, n-Propyl oder Methoxymethyl,
m bedeutet 0 oder 1.

Erfindungsgemäße Verbindungen, in denen Q für Q1 oder Q2 steht, können beispielsweise nach der in Schema 1 angegebenen Methode durch basenkatalysierte Umsetzung eines Benzoesäurechlorids (II) mit einem 5-Amino-1-H-1,2,4-triazol bzw. 5-Amino-1H-tetrazol (III) hergestellt werden:

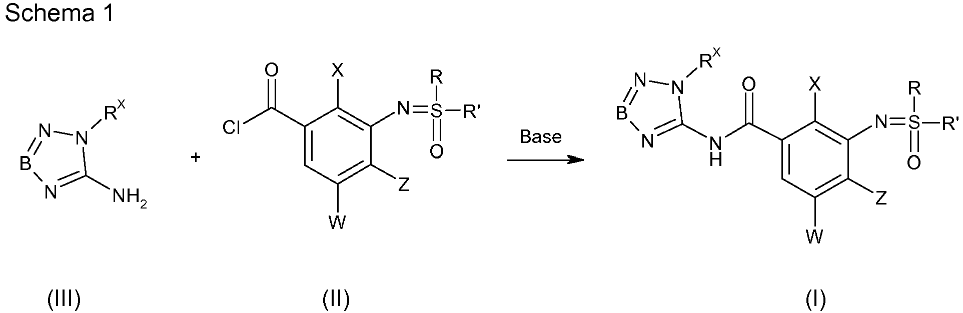

Darin steht B für CH oder N.

Die Benzoesäurechloride der Formel (II) beziehungsweise die ihnen zugrunde liegenden Benzoesäuren sind grundsätzlich bekannt und können beispielsweise gemäß den in WO 2004/052849 A1, WO 2008/035737 A1, WO 2009/116290 A1, WO 2010/016230 A1 und US 2011/0144345 A1 beschriebenen Methoden hergestellt werden.

Erfindungsgemäße Verbindungen, in denen Q für Q1 oder Q2 steht, können auch nach der in Schema 2 angegebenen Methode durch Umsetzung einer Benzoesäure der Formel (IV) mit einem 5-Amino-1-H-1,2,4-triazol bzw. 5-Amino-1H-tetrazol (III) hergestellt werden:

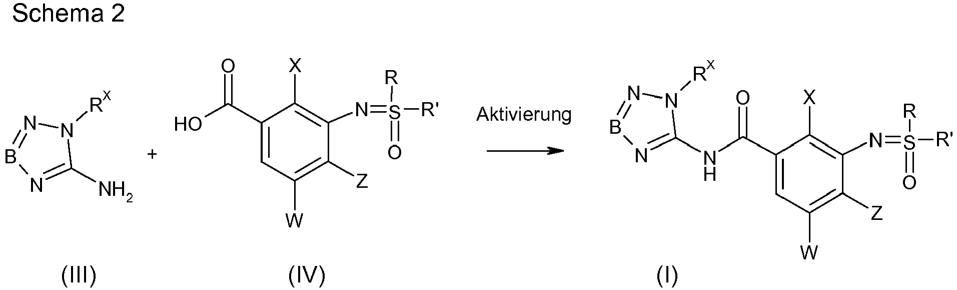

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) eingesetzt werden.

Erfindungsgemäße Verbindungen, in denen Q für Q1 oder Q2 steht, können auch nach der in Schema 3 angegebenen Methode durch Umsetzung eines N-(1H-1,2,4-triazol-5-yl)benzamids oder eines N-(1H-tetrazol-5-yl)benzamids hergestellt werden:

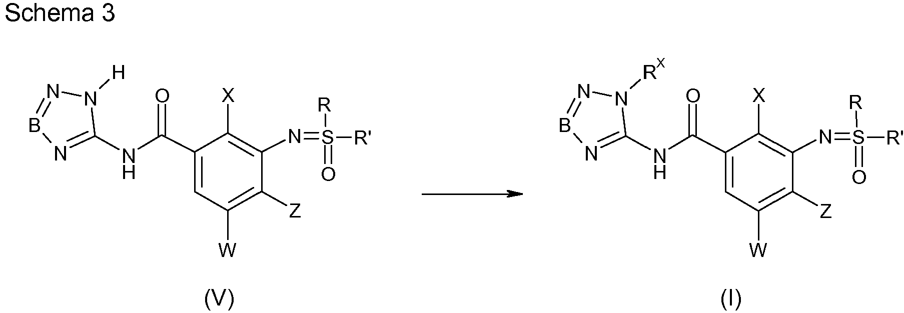

Für diese in Schema 3 genannte Reaktion können Alkylierungsmittel wie Alkylhalogenide, -sulfonate oder Dialkylsulfate in Gegenwart einer Base eingesetzt werden.

Die 5-Amino-1H-tetrazole der Formel (III) sind entweder käuflich erhältlich oder können analog zu literaturbekannten Methoden hergestellt werden. Beispielsweise können substituierte 5-Aminotetrazole nach der in Journal of the American Chemical Society (1954), 76, 923-924 beschriebenen Methode aus Amino-tetrazol hergestellt werden:

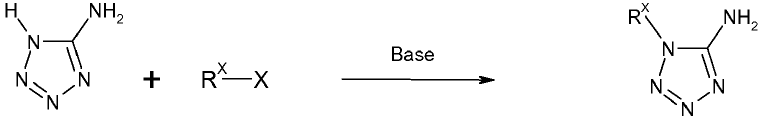

In der vorstehend genannten Reaktion bedeutet X eine Abgangsgruppe wie Iod. Substituierte 5-Aminotetrazole können zum Beispiel auch wie in Journal of the American Chemical Society (1954) 76, 88-89 beschrieben, synthetisiert werden:

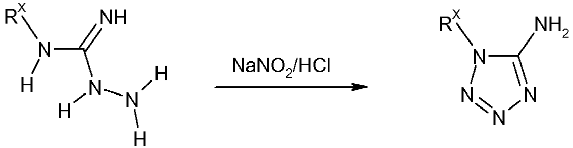

Die 5-Amino-1H-triazole der Formel (III) sind entweder käuflich erhältlich oder können analog zu literaturbekannten Methoden hergestellt werden. Beispielsweise können substituierte 5-Aminotriazole nach der in Zeitschrift für Chemie (1990), 30(12), 436 - 437 beschriebenen Methode aus Aminotriazol hergestellt werden:

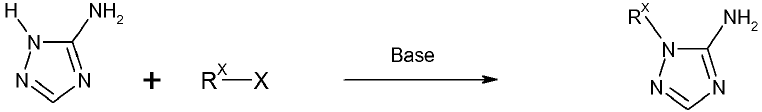

Substituierte 5-Aminotriazole können auch zum Beispiel wie in Chemische Berichte (1964), 97(2), 396-404 beschrieben synthetisiert werden:

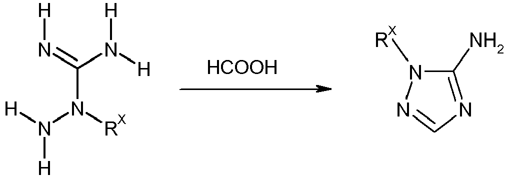

Substituierte 5-Aminotriazole können auch zum Beispiel wie in Angewandte Chemie (1963), 75, 918 beschrieben, synthetisiert werden:

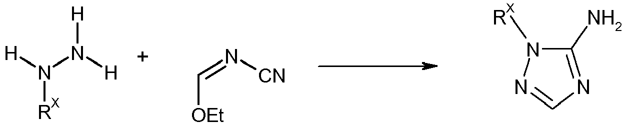

Erfindungsgemäße Verbindungen, in denen Q für Q3 steht, können beispielsweise nach der in Schema 4 angegebenen Methode durch basenkatalysierte Umsetzung eines Benzoesäurechlorids (II) mit einem 4-Amino-1,2,5-oxadiazol (VI) hergestellt werden:

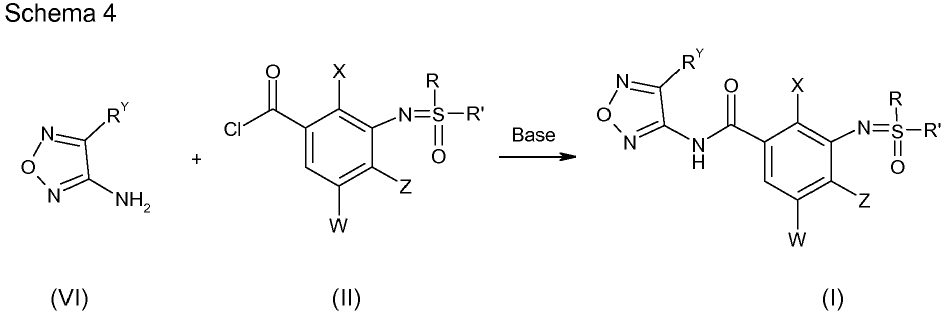

Erfindungsgemäße Verbindungen können auch nach der in Schema 5 angegebenen Methode durch Umsetzung einer Benzoesäure der Formel (IV) mit einem 4-Amino-1,2,5-oxadiazol (VI) hergestellt werden:

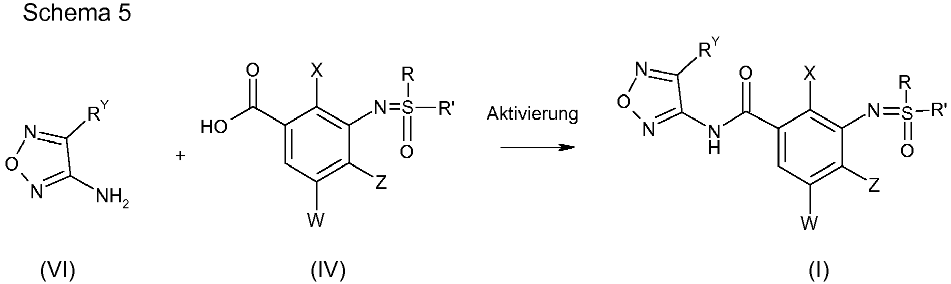

Für die Aktivierung können wasserentziehende Reagenzien die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc., eingesetzt werden.

Die 4-Amino-1,2,5-oxadiazole der Formel (VI) sind entweder käuflich erhältlich oder bekannt oder können analog zu literaturbekannten Methoden hergestellt werden. Beispielsweise können 3-Alkyl-4-amino-1,2,5-oxadiazole nach der in Russian Chemical Bulletin, Int. Ed., Vol. 54, No. 4, S. 1032-1037 (2005) beschriebenen Methode aus ß-Ketoestern hergestellt werden:

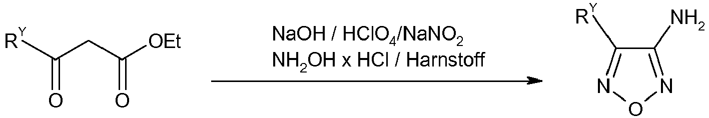

3-Aryl-4-amino-1,2,5-oxadiazole können zum Beispiel wie in Russian Chemical Bulletin, 54(4), 1057-1059, (2005) oder Indian Journal of Chemistry, Section B: Organic Chemistry Including Medicinal Chemistry, 26B(7), 690-2, (1987) beschrieben, synthetisiert werden:

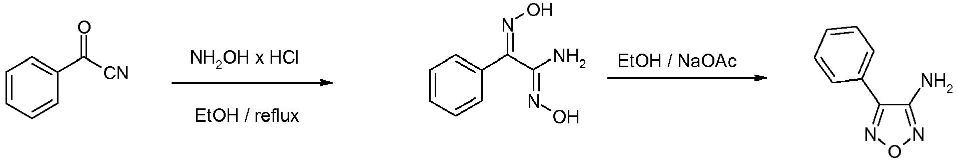

3-Amino-4-Halogen-1,2,5-oxadiazole können beispielsweise nach der in Heteroatom Chemistry 15(3), 199-207 (2004) beschrieben Methode aus dem käuflich erhältlichen 3,4-Diamino-1,2,5-oxadiazol durch eine Sandmeyer-Reaktion hergestellt werden:

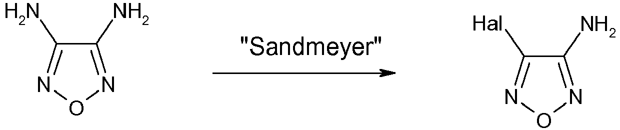

Nucleophile Reste R^{Y} können wie in Journal of Chemical Research, Synopses, (6), 190, 1985 oder in Izvestiya Akademii Nauk SSSR, Seriya Khimicheskaya, (9), 2086-8, 1986 oder in Russian Chemical Bulletin (Translation of Izvestiya Akademii Nauk, Seriya Khimicheskaya), 53(3), 596-614, 2004 beschrieben, durch Substitution der Austrittsgruppe L in 3-Amino-1,2,5-oxadiazolen eingeführt werden. L steht für eine Abgangsgruppe wie z. B. Chlor, Brom, Jod, Mesyloxy, Tosyloxy, Trifluorsulfonyloxy etc.

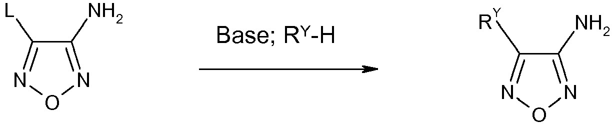

Erfindungsgemäße Verbindungen, in denen Q für Q4 steht, können beispielsweise nach der in Schema 6 angegebenen Methode durch basenkatalysierte Umsetzung eines Benzoesäurechlorids (II) mit einem 2-Amino-1,3,4-oxadiazol (VII) hergestellt werden:

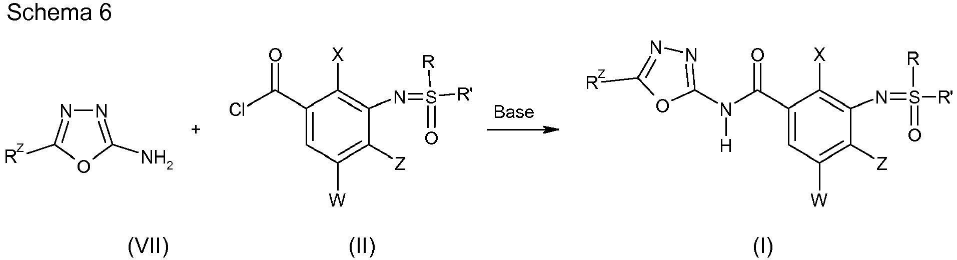

Erfindungsgemäße Verbindungen können auch nach der in Schema 7 angegebenen Methode durch Umsetzung einer Benzoesäure der Formel (IV) mit einem 2-Amino-1,3,4-oxadiazol (VII) hergestellt werden:

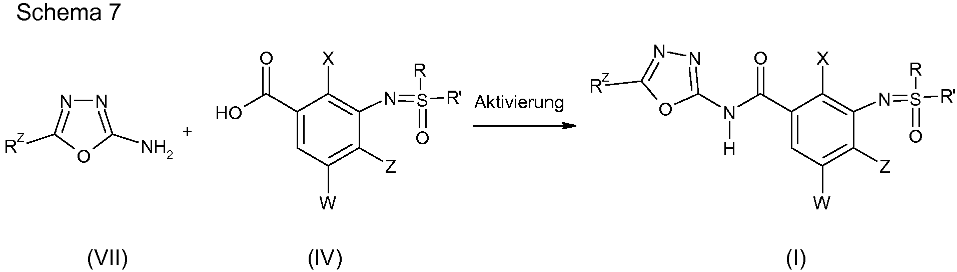

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen können auch nach der in Schema 8 angegebenen Methode durch Cyclisierung einer Verbindung der Formel (VIII) hergestellt werden:

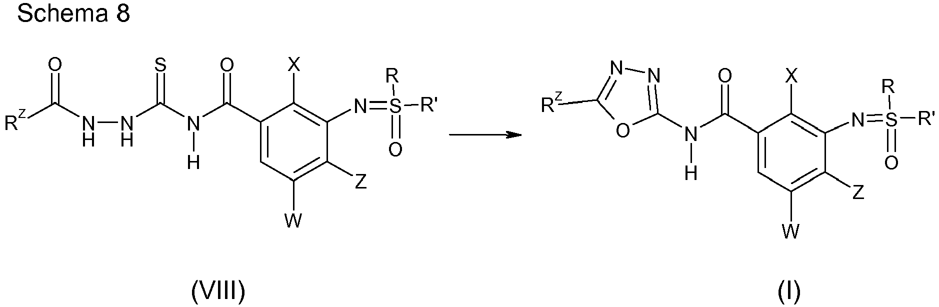

Die Cyclisierung kann gemäß der in Synth. Commun. 31 (12), 1907-1912 (2001) oder der in Indian J. Chem., Section B: Organic Chemistry Including Medicinal Chemistry; Vol. 43 (10), 2170-2174 (2004) beschriebenen Methoden durchgeführt werden.

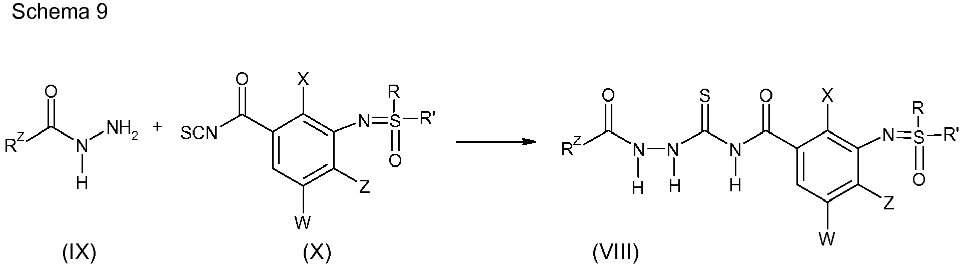

Die in Schema 8 eingesetzte Verbindung der Formel (VIII) kann durch Umsetzung eines Acylisothiocyanats der Formel (X) mit einem Hydrazid der Formel (IX) gemäß der in Synth. Commun. 25(12), 1885-1892 (1995) beschriebenen Methode hergestellt werden.

Erfindungsgemäße Verbindungen, in denen der Substituent R" nicht Wasserstoff bedeutet, können beispielsweise nach der in Schema 10 angegebenen Methode durch Umsetzung eines N-(1,2,5-Oxadiazol-3-yl)-, N-(1,3,4-Oxadiazol-2-yl), N-(Tetrazol-5-yl)- oder N-(Triazol-5-yl)- arylcarbonsäureamids (I) mit einer Verbindung der allgemeinen Formel (XI), wobei L für eine Abgangsgruppe wie z. B. Chlor, Brom, Jod, Mesyloxy, Tosyloxy, Trifluorsulfonyloxy etc. steht, hergestellt werden:

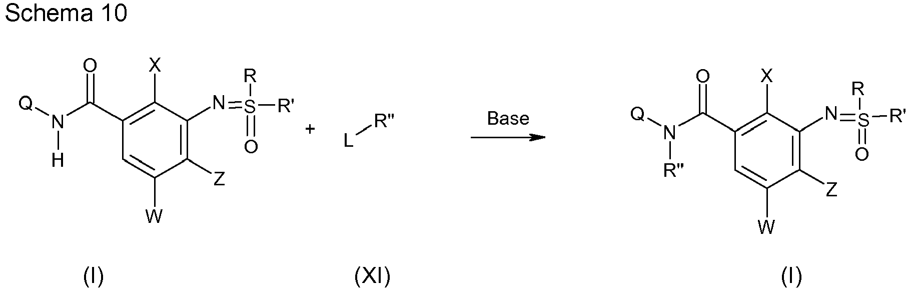

Die Verbindungen der Formel (XI) sind entweder käuflich oder können nach bekannten in der Literatur beschriebenen Methoden hergestellt werden.

Erfindungsgemäße Verbindungen können auch nach der in Schema 11 angegebenen Methode durch Umsetzung eines Amins der Formel (XII) mit einem Säurechlorid (II), wie zum Beispiel in J. Het: Chem. (1972), 9 (1), 107-109) beschrieben, hergestellt werden:

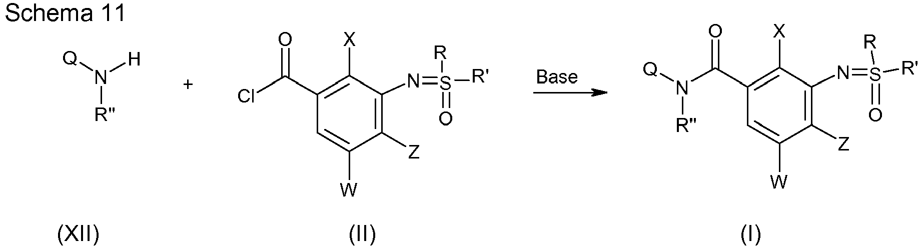

Erfindungsgemäße Verbindungen können auch nach der in Schema 12 angegebenen Methode durch Umsetzung eines Amins der Formel (XII) mit einer Säure der Formel (IV) hergestellt werden:

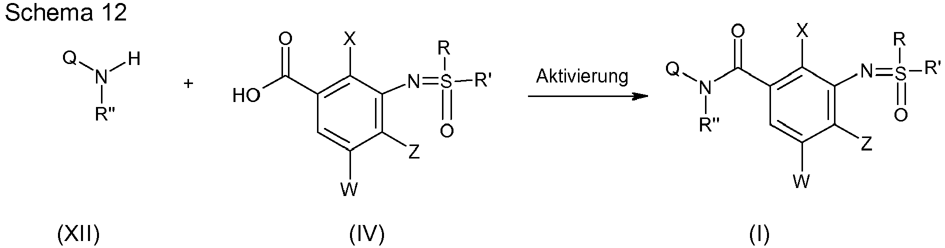

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Die Amine der Formel (XII) sind entweder käuflich oder in der Literatur bekannt oder können beispielsweise nach der in Schema 13 beschriebenen Methoden durch basenkatalysierte Alkylierung oder durch reduktive Aminierung oder nach der in Schema 14 beschriebenen Methode durch nucleophile Substitution einer Abgangsgruppe L durch Amine R"-NH₂ hergestellt werden, wobei L für eine Abgangsgruppe wie z. B. Chlor, Brom, Jod, Mesyloxy, Tosyloxy, Trifluorsulfonyloxy etc. steht.

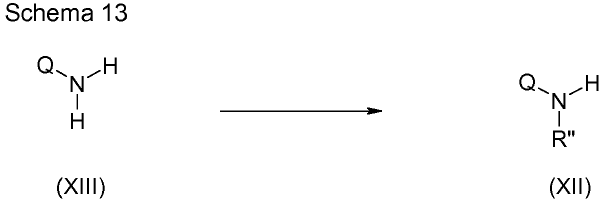

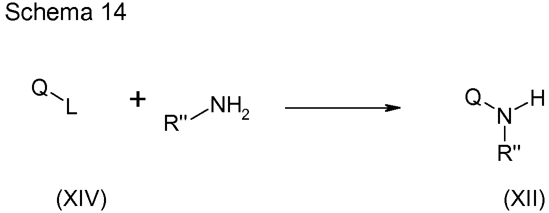

Die Amine der Formel (XII) können auch durch Cyclisierungsreaktionen wie zum Beispiel in J. Org. Chem. 73(10), 3738-3744 (2008) für Q = Q1 oder in Buletinul Institutului Politehnic din lasi (1974), 20(1-2), 95-99 oder in J. Org. Chem. 67(21), 7361-7364 (2002) für Q = Q4 beschrieben, hergestellt werden.

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren. Es ist möglicherweise von Vorteil, das Sulfoximin erst am Ende der Synthesesequenz auf der Benzamid-Stufe zu generieren.

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Synthese von 2,4-Dichlor-3-{[diethyl(oxido)-λ4-sulfanyliden]amino}-N-(1-methyltetrazol-5-yl)-benzamid (Tabellenbeispiel Nr. 1-14)

### Schritt 1: Synthese von 2,4-Dichlor-3-(nonafluor-n-butylsulfonyloxy)benzoesäuremethylester

9.5 g (43.0 mmol) 2,4-Dichlor-3-hydroxybenzoesäuremethylester wurden in 240 ml Acetonitril mit 8.6 g (62.2 mmol) Kaliumcarbonat und danach mit 9.5 ml (52.8 mmol) Nonafluor-n-butansulfonsäurechlorid versetzt. Die Reaktionsmischung wurde 16 h bei Raumtemperatur (RT) gerührt. Zur Aufarbeitung wurde der Inhalt auf Eiswasser gegossen und die Mischung wurde mit Essigsäureethylester extrahiert. Die organische Phase wurde getrocknet und das Filtrat wurde am Rotationsverdampfer vom Lösungsmittel befreit. Der Rückstand wurde chromatographisch gereinigt, wobei 3.5 g sauberes Produkt erhalten wurden.

### Schritt 2: Synthese von 2,4-Dichlor-3-{[diethyl(oxido)-λ4-sulfanyliden]amino}benzoesäuremethylester

9.5 g (18.9 mmol) 2,4-Dichlor-3-(nonafluor-n-butylsulfonyloxy)benzoesäuremethylester wurden in 100 ml Toluol gelöst. Die Lösung wurde unter Stickstoffatmosphäre mit Stickstoff 5 - 10 Minuten gespült. Anschließend wurden nacheinander 2.83 g (23.3 mmol) S,S-Diethylsulfoximin, 0.19 g (0.846 mmol) Palladium(II)acetat, 0.84 g (1.35 mmol) (+/-)-2,2'-Bis(diphenylphosphino)-1,1'-binaphthyl sowie 9.8 g (30.1 mmol) Cäsiumcarbonat zugegeben. Anschließend wurde die Mischung nochmals 15 -20 Minuten mit Stickstoff gespült. Die Reaktionsmischung wurde 4 h lang unter Rückfluss erhitzt. Zur Aufarbeitung wurde der Inhalt auf RT abgekühlt, auf Eiswasser gegossen und anschließend wurde die Mischung mit Essigsäureethylester extrahiert. Die organische Phase wurde getrocknet und unter Vakuum destilliert. Das gewonnene Rohprodukt wurde chromatographisch gereinigt, wobei 4.5 g sauberes Produkt isoliert wurden.

### Schritt 3: Synthese von 2,4-Dichlor-3-{[diethyl(oxido)-λ4-sulfanyliden]amino}benzoesäure

4.5 g (13.9 mmol) 2,4-Dichlor-3-{[diethyl(oxido)-λ4-sulfanyliden]amino}benzoesäuremethylester wurden in 160 ml Ethanol gelöst. Anschließend wurden 16.8 ml (42 mmol) einer wässrigen, 10 %-igen Natronlauge tropfenweise zugegeben. Der Inhalt wurde danach 3 h bei RT gerührt. Zur Aufarbeitung wurde das Ethanol unter Vakuum am Rotationsverdampfer entfernt. Der Rückstand wurde mit Wasser versetzt und die Mischung wurde mit Diethylether gewaschen. Die wässrige Phase wurde mit konzentrierter Salzsäure angesäuert und mit Essigsäureethylester extrahiert. Die organische Phase wurde getrocknet und das Filtrat wurde am Rotationsverdampfer vom Lösungsmittel befreit. Als Rückstand wurden 3.5 g des Produkts isoliert.

### Schritt 4: Synthese von 2,4-Dichlor-3-{[diethyl(oxido)-λ4-sulfanyliden]amino}-N-(1-methyltetrazol-5-yl)-benzamid

250 mg (0.81 mmol) 2,4-Dichlor-3-{[diethyl(oxido)-λ4-sulfanyliden]amino}benzoesäure und 112 mg (1.13 mmol) 5-Amino-1-methyl-1H-tetrazol wurden in 7.5 ml Pyridin mit 133 mg (1.05 mmol) Oxalsäuredichlorid versetzt und dann drei Tage bei RT gerührt. Zur Aufarbeitung wurde eingeengt und der Rückstand wurde mit CH₂Cl₂ und einer gesättigten wässrigen Natriumhydrogencarbonatlösung gerührt. Die organische Phase wurde erneut eingeengt und der Rückstand chromatographisch gereinigt, wobei 170 mg sauberes Produkt erhalten wurden.

### Synthese von 2,4-Dichlor-3-[(4-oxido-1,4-λ4-oxathian-4-yliden)amino]-N-(4-methyl-1,2,5-oxadiazol-3-yl)-benzamid (Tabellenbeispiel Nr. 6-102)

### Schritt 1: Synthese von 2,4-Dichlor-3-[(4-oxido-1,4-λ4-oxathian-4-yliden)amino]-benzoesäuremethylester

5 g (9.94 mmol) 2,4-Dichlor-3-(nonafluor-n-butylsulfonyloxy)benzoesäuremethylester wurden in 100 ml Toluol gelöst. Die Lösung wurde unter Stickstoffatmosphäre mit Stickstoff 5 - 10 Minuten gespült. Anschließend wurden nacheinander 1.6 g (11.9 mmol) 2H-4λ4-1,4-Oxathiin-4-imin-4-oxid, 0.1 g (0.46 mmol) Palladium(II)acetat, 0.43 g (0.7 mmol) (+/-)-2,2'-Bis(diphenylphosphino)-1,1'-binaphthyl sowie 4.8 g (14 mmol) Cäsiumcarbonat zugegeben. Anschließend wurde die Mischung nochmals 15 -20 Minuten mit Stickstoff gespült. Die Reaktionsmischung wurde 4 h lang unter Rückfluss erhitzt. Zur Aufarbeitung wurde der Inhalt auf RT abgekühlt, auf Eiswasser gegossen und anschließend wurde die Mischung mit CH₂Cl₂ extrahiert. Die organische Phase wurde getrocknet und unter Vakuum destilliert. Das gewonnene Rohprodukt wurde chromatographisch gereinigt, wobei 1.8 g sauberes Produkt isoliert wurden.

### Schritt 2: Synthese von 2,4-Dichlor-3-[(4-oxido-1,4-λ4-oxathian-4-yliden)amino]-benzoesäure

1.8 g (5.32 mmol) 2,4-Dichlor-3-[(4-oxido-1,4-λ4-oxathian-4-yliden)amino]benzoesäuremethylester wurden in 40 ml Methanol gelöst. Anschließend wurden 31 ml (77.5 mmol) einer wässrigen, 10 %-igen Natronlauge zugegeben. Der Inhalt wurde danach 1 h bei RT gerührt. Zur Aufarbeitung wurde das Methanol unter Vakuum am Rotationsverdampfer entfernt. Der Rückstand wurde mit Wasser versetzt und die Mischung wurde mit Diethylether gewaschen. Die wässrige Phase wurde mit 1 M Salzsäure angesäuert und mit Essigsäureethylester extrahiert. Die organische Phase wurde getrocknet und das Filtrat wurde am Rotationsverdampfer vom Lösungsmittel befreit. Als Rückstand wurden 1.2 g des Produkts isoliert.

### Schritt 3: Synthese von 2,4-Dichlor-3-[(4-oxido-1,4-λ4-oxathian-4-yliden)amino]-N-(4-methyl-1,2,5-oxadiazol-3-yl)-benzamid

200 mg (0.62 mmol) 2,4-Dichlor-3-[(4-oxido-1,4-λ4-oxathian-4-yliden)amino]-benzoesäure und 67.2 mg (0.68 mmol) 4-Methyl-1,2,5-oxadiazol-3-yl-amin wurden in 15 ml CH₂Cl₂ mit 589 mg (0.93 mmol; 50%ige Lösung in THF) 2,4,6-Tripropyl-1,3,5,2,4,6-trioxatriphosphinan-2,4,6-trioxid versetzt. Das Gemisch wurde 1 h bei RT gerührt. Anschließend wurden 312 mg (3.09 mmol) NEt₃ tropfenweise zugegeben, danach eine katalytische Menge 4-(Dimethylamino)pyridin. Der Inhalt wurde drei Tage bei RT gerührt. Zur Aufarbeitung wurde das Gemisch mit 1 M Salzsäure gewaschen. Nach der Phasentrennung wurde der Inhalt eingeengt und der Rückstand wurde chromatographisch gereinigt, wobei 90 mg sauberes Produkt erhalten wurden.

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt

Die verwendeten Abkürzungen bedeuten:
Et = Ethyl Me = Methyl n-Pr = n-Propyl i-Pr = Isopropyl c-Pr = Cyclopropyl Ph = Phenyl

**Tabelle 1: Erfindungsgemäße Verbindungen der allgemeinen Formel (I), worin Q für Q1 und R^{x} für eine Methylgruppe stehen sowie R" und W jeweils Wasserstoff bedeuten**

| | | | | | |
|---|---|---|---|---|---|
| 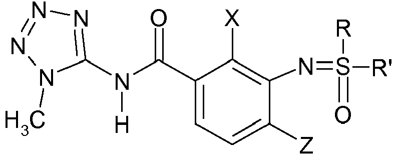 | | | | | |

| Nr. | X | Z | R | R' | Physikalische Daten (¹H-NMR) |
|---|---|---|---|---|---|
| 1-1 | Me | Me | Et | Et | |
| 1-2 | Me | F | Et | Et | |
| 1-3 | Me | Cl | Et | Et | |
| 1-4 | Me | Br | Et | Et | |
| 1-5 | Me | I | Et | Et | |
| 1-6 | Me | CF₃ | Et | Et | |
| 1-7 | Me | CHF₂ | Et | Et | |
| 1-8 | Me | CF₂Cl | Et | Et | |
| 1-9 | Me | OMe | Et | Et | |
| 1-10 | Me | NO₂ | Et | Et | |
| 1-11 | Me | SO₂Me | Et | Et | |
| 1-12 | Cl | Me | Et | Et | |
| 1-13 | Cl | F | Et | Et | |
| 1-14 | Cl | Cl | Et | Et | (400 MHz, DMSO-d₆ δ, ppm) 7.54 (d,1H), 7.30 (d,1H), 3.99 (s,3H), 3.42 - 3.26 (m,4H), 1.33 (t,6H) |
| 1-15 | Cl | Br | Et | Et | |
| 1-16 | Cl | I | Et | Et | |
| 1-17 | Cl | CF₃ | Et | Et | |
| 1-18 | Cl | CHF₂ | Et | Et | |
| 1-19 | Cl | CF₂Cl | Et | Et | |
| 1-20 | Cl | OMe | Et | Et | |
| 1-21 | Cl | NO₂ | Et | Et | |
| 1-22 | Cl | SO₂Me | Et | Et | |
| 1-23 | OMe | Me | Et | Et | |
| 1-24 | OMe | F | Et | Et | |
| 1-25 | OMe | Cl | Et | Et | |
| 1-26 | OMe | Br | Et | Et | |
| 1-27 | OMe | I | Et | Et | |
| 1-28 | OMe | CF₃ | Et | Et | |
| 1-29 | OMe | CHF₂ | Et | Et | |
| 1-30 | OMe | CF₂Cl | Et | Et | |
| 1-31 | OMe | OMe | Et | Et | |
| 1-32 | OMe | NO₂ | Et | Et | |
| 1-33 | OMe | SO₂Me | Et | Et | |
| 1-34 | SO₂Me | Me | Et | Et | |
| 1-35 | SO₂Me | F | Et | Et | |
| 1-36 | SO₂Me | Cl | Et | Et | |
| 1-37 | SO₂Me | Br | Et | Et | |
| 1-38 | SO₂Me | I | Et | Et | |
| 1-39 | SO₂Me | CF₃ | Et | Et | |
| 1-40 | SO₂Me | CHF₂ | Et | Et | |
| 1-41 | SO₂Me | CF₂Cl | Et | Et | |
| 1-42 | SO₂Me | OMe | Et | Et | |
| 1-43 | SO₂Me | NO₂ | Et | Et | |
| 1-44 | SO₂Me | SO₂Me | Et | Et | |
| 1-45 | Me | Me | - (CH₂)₅ - | | |
| 1-46 | Me | F | - (CH₂)₅ - | | |
| 1-47 | Me | Cl | - (CH₂)₅ - | | |
| 1-48 | Me | Br | - (CH₂)₅ - | | |
| 1-49 | Me | I | - (CH₂)₅ - | | |
| 1-50 | Me | CF₃ | - (CH₂)₅ - | | |
| 1-51 | Me | CHF₂ | - (CH₂)₅ - | | |
| 1-52 | Me | CF₂Cl | -(CH₂)₅- | | |
| 1-53 | Me | OMe | -(CH₂)₅- | | |
| 1-54 | Me | NO₂ | -(CH₂)₅- | | |
| 1-55 | Me | SO₂Me | -(CH₂)₅- | | |
| 1-56 | Cl | Me | -(CH₂)₅- | | |
| 1-57 | Cl | F | -(CH₂)₅- | | |
| 1-58 | Cl | Cl | -(CH₂)₅- | | (400 MHz, DMSO-d₆ δ, ppm) 7.55 (d,1H), 7.31 (d,1H), 3.99 (s,3H), 3.42 - 3.25 (m,4H), 2.13 - 2.05 (m,2H), 1.98 - 1.88 (m,2H), 1.70 - 1.55 (m,2H) |
| 1-59 | Cl | Br | -(CH₂)₅- | | |
| 1-60 | Cl | I | -(CH₂)₅- | | |
| 1-61 | Cl | CF₃ | -(CH₂)₅- | | |
| 1-62 | Cl | CHF₂ | -(CH₂)₅- | | |
| 1-63 | Cl | CF₂Cl | -(CH₂)₅- | | |
| 1-64 | Cl | OMe | -(CH₂)₅- | | |
| 1-65 | Cl | NO₂ | -(CH₂)₅- | | |
| 1-66 | Cl | SO₂Me | -(CH₂)₅- | | |
| 1-67 | OMe | Me | -(CH₂)₅- | | |
| 1-68 | OMe | F | -(CH₂)₅- | | |
| 1-69 | OMe | Cl | -(CH₂)₅- | | |
| 1-70 | OMe | Br | -(CH₂)₅- | | |
| 1-71 | OMe | I | -(CH₂)₅- | | |
| 1-72 | OMe | CF₃ | -(CH₂)₅- | | |
| 1-73 | OMe | CHF₂ | -(CH₂)₅- | | |
| 1-74 | OMe | CF₂Cl | -(CH₂)₅- | | |
| 1-75 | OMe | OMe | -(CH₂)₅- | | |
| 1-76 | OMe | NO₂ | -(CH₂)₅- | | |
| 1-77 | OMe | SO₂Me | -(CH₂)₅- | | |
| 1-78 | SO₂Me | Me | -(CH₂)₅- | | |
| 1-79 | SO₂Me | F | -(CH₂)₅- | | |
| 1-80 | SO₂Me | Cl | -(CH₂)₅- | | |
| 1-81 | SO₂Me | Br | -(CH₂)₅- | | |
| 1-82 | SO₂Me | I | -(CH₂)₅- | | |
| 1-83 | SO₂Me | CF₃ | -(CH₂)₅- | | |
| 1-84 | SO₂Me | CHF₂ | -(CH₂)₅- | | |
| 1-85 | SO₂Me | CF₂Cl | -(CH₂)₅- | | |
| 1-86 | SO₂Me | OMe | -(CH₂)₅- | | |
| 1-87 | SO₂Me | NO₂ | -(CH₂)₅- | | |
| 1-88 | SO₂Me | SO₂Me | -(CH₂)₅- | | |
| 1-89 | Me | Me | -(CH₂)₂O(CH₂)₂- | | |
| 1-90 | Me | F | -(CH₂)₂O(CH₂)₂- | | |
| 1-91 | Me | Cl | -(CH₂)₂O(CH₂)₂- | | |
| 1-92 | Me | Br | -(CH₂)₂O(CH₂)₂- | | |
| 1-93 | Me | I | -(CH₂)₂O(CH₂)₂- | | |
| 1-94 | Me | CF₃ | -(CH₂)₂O(CH₂)₂- | | |
| 1-95 | Me | CHF₂ | -(CH₂)₂O(CH₂)₂- | | |
| 1-96 | Me | CF₂Cl | -(CH₂)₂O(CH₂)₂- | | |
| 1-97 | Me | OMe | -(CH₂)₂O(CH₂)₂- | | |
| 1-98 | Me | NO₂ | -(CH₂)₂O(CH₂)₂- | | |
| 1-99 | Me | SO₂Me | -(CH₂)₂O(CH₂)₂- | | |
| 1-100 | Cl | Me | -(CH₂)₂O(CH₂)₂- | | |
| 1-101 | Cl | F | -(CH₂)₂O(CH₂)₂- | | |
| 1-102 | Cl | Cl | -(CH₂)₂O(CH₂)₂- | | (400 MHz, DMSO-d₆ δ, ppm) 7.55 (d,1H), 7.32 (d,1H), 4.20 (m,2H), 4.02 (m,2H), 3.95 (s,3H), 3.55 - 3.37 (m,4H) |
| 1-103 | Cl | Br | -(CH₂)₂O(CH₂)₂- | | |
| 1-104 | Cl | I | -(CH₂)₂O(CH₂)₂- | | |
| 1-105 | Cl | CF₃ | -(CH₂)₂O(CH₂)₂- | | |
| 1-106 | Cl | CHF₂ | -(CH₂)₂O(CH₂)₂- | | |
| 1-107 | Cl | CF₂Cl | -(CH₂)₂O(CH₂)₂- | | |
| 1-108 | Cl | OMe | -(CH₂)₂O(CH₂)₂- | | |
| 1-109 | Cl | NO₂ | -(CH₂)₂O(CH₂)₂- | | |
| 1-110 | Cl | SO₂Me | -(CH₂)₂O(CH₂)₂- | | |
| 1-111 | OMe | Me | -(CH₂)₂O(CH₂)₂- | | |
| 1-112 | OMe | F | -(CH₂)₂O(CH₂)₂- | | |
| 1-113 | OMe | Cl | -(CH₂)₂O(CH₂)₂- | | |
| 1-114 | OMe | Br | -(CH₂)₂O(CH₂)₂- | | |
| 1-115 | OMe | I | -(CH₂)₂O(CH₂)₂- | | |
| 1-116 | OMe | CF₃ | -(CH₂)₂O(CH₂)₂- | | |
| 1-117 | OMe | CHF₂ | -(CH₂)₂O(CH₂)₂- | | |
| 1-118 | OMe | CF₂Cl | -(CH₂)₂O(CH₂)₂- | | |
| 1-119 | OMe | OMe | -(CH₂)₂O(CH₂)₂- | | |
| 1-120 | OMe | NO₂ | -(CH₂)₂O(CH₂)₂- | | |
| 1-121 | OMe | SO₂Me | -(CH₂)₂O(CH₂)₂- | | |
| 1-122 | SO₂Me | Me | -(CH₂)₂O(CH₂)₂- | | |
| 1-123 | SO₂Me | F | -(CH₂)₂O(CH₂)₂- | | |
| 1-124 | SO₂Me | Cl | -(CH₂)₂O(CH₂)₂- | | |
| 1-125 | SO₂Me | Br | -(CH₂)₂O(CH₂)₂- | | |
| 1-126 | SO₂Me | I | -(CH₂)₂O(CH₂)₂- | | |
| 1-127 | SO₂Me | CF₃ | -(CH₂)₂O(CH₂)₂- | | |
| 1-128 | SO₂Me | CHF₂ | -(CH₂)₂O(CH₂)₂- | | |
| 1-129 | SO₂Me | CF₂Cl | -(CH₂)₂O(CH₂)₂- | | |
| 1-130 | SO₂Me | OMe | -(CH₂)₂O(CH₂)₂- | | |
| 1-131 | SO₂Me | NO₂ | -(CH₂)₂O(CH₂)₂- | | |
| 1-132 | SO₂Me | SO₂Me | -(CH₂)₂O(CH₂)₂- | | |
| 1-133 | Cl | COOMe | Et | Et | |
| 1-134 | Cl | COOMe | -(CH₂)₅- | | |
| 1-135 | Cl | COOMe | -(CH₂)₂O(CH₂)₂- | | |

**Tabelle 2: Erfindungsgemäße Verbindungen der allgemeinen Formel (I), worin Q für Q1 und R^{x} für eine Ethylgruppe stehen sowie R" und W jeweils Wasserstoff bedeuten**

| | | | | | |
|---|---|---|---|---|---|
| 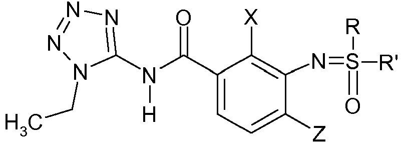 | | | | | |

| Nr. | X | Z | R | R' | Physikalische Daten (¹H-NMR) |
|---|---|---|---|---|---|
| 2-1 | Me | Me | Et | Et | |
| 2-2 | Me | F | Et | Et | |
| 2-3 | Me | Cl | Et | Et | |
| 2-4 | Me | Br | Et | Et | |
| 2-5 | Me | I | Et | Et | |
| 2-6 | Me | CF₃ | Et | Et | |
| 2-7 | Me | CHF₂ | Et | Et | |
| 2-8 | Me | CF₂Cl | Et | Et | |
| 2-9 | Me | OMe | Et | Et | |
| 2-10 | Me | NO₂ | Et | Et | |
| 2-11 | Me | SO₂Me | Et | Et | |
| 2-12 | Cl | Me | Et | Et | |
| 2-13 | Cl | F | Et | Et | |
| 2-14 | Cl | Cl | Et | Et | (400 MHz, DMSO-d₆ δ, ppm) 7.53 (d,1H), 7.28 (d,1H), 4.34 (q,2H), 1.46 (t,3H), 1.33 (t,6H) |
| 2-15 | Cl | Br | Et | Et | |
| 2-16 | Cl | I | Et | Et | |
| 2-17 | Cl | CF₃ | Et | Et | |
| 2-18 | Cl | CHF₂ | Et | Et | |
| 2-19 | Cl | CF₂Cl | Et | Et | |
| 2-20 | Cl | OMe | Et | Et | |
| 2-21 | Cl | NO₂ | Et | Et | |
| 2-22 | Cl | SO₂Me | Et | Et | |
| 2-23 | OMe | Me | Et | Et | |
| 2-24 | OMe | F | Et | Et | |
| 2-25 | OMe | Cl | Et | Et | |
| 2-26 | OMe | Br | Et | Et | |
| 2-27 | OMe | I | Et | Et | |
| 2-28 | OMe | CF₃ | Et | Et | |
| 2-29 | OMe | CHF₂ | Et | Et | |
| 2-30 | OMe | CF₂Cl | Et | Et | |
| 2-31 | OMe | OMe | Et | Et | |
| 2-32 | OMe | NO₂ | Et | Et | |
| 2-33 | OMe | SO₂Me | Et | Et | |
| 2-34 | SO₂Me | Me | Et | Et | |
| 2-35 | SO₂Me | F | Et | Et | |
| 2-36 | SO₂Me | Cl | Et | Et | |
| 2-37 | SO₂Me | Br | Et | Et | |
| 2-38 | SO₂Me | I | Et | Et | |
| 2-39 | SO₂Me | CF₃ | Et | Et | |
| 2-40 | SO₂Me | CHF₂ | Et | Et | |
| 2-41 | SO₂Me | CF₂Cl | Et | Et | |
| 2-42 | SO₂Me | OMe | Et | Et | |
| 2-43 | SO₂Me | NO₂ | Et | Et | |
| 2-44 | SO₂Me | SO₂Me | Et | Et | |
| 2-45 | Me | Me | -(CH₂)₅- | | |
| 2-46 | Me | F | -(CH₂)₅- | | |
| 2-47 | Me | Cl | -(CH₂)₅- | | |
| 2-48 | Me | Br | -(CH₂)₅- | | |
| 2-49 | Me | I | -(CH₂)₅- | | |
| 2-50 | Me | CF₃ | -(CH₂)₅- | | |
| 2-51 | Me | CHF₂ | -(CH₂)₅- | | |
| 2-52 | Me | CF₂Cl | -(CH₂)₅- | | |
| 2-53 | Me | OMe | -(CH₂)₅- | | |
| 2-54 | Me | NO₂ | -(CH₂)₅- | | |
| 2-55 | Me | SO₂Me | -(CH₂)₅- | | |
| 2-56 | Cl | Me | -(CH₂)₅- | | |
| 2-57 | Cl | F | -(CH₂)₅- | | |
| 2-58 | Cl | Cl | -(CH₂)₅- | | (400 MHz, DMSO-d₆ δ, ppm) 7.50 (d,1H), 7.24 (d, 1H), 4.30 (q,2H), 2.12 - 2.03 (m,2H), 2.00 - 1.88 (m,4H), 1.70 - 1.53 (m,2H), 1.43 (t,3H) |
| 2-59 | Cl | Br | -(CH₂)₅- | | |
| 2-60 | Cl | I | -(CH₂)₅- | | |
| 2-61 | Cl | CF₃ | -(CH₂)₅- | | |
| 2-62 | Cl | CHF₂ | -(CH₂)₅- | | |
| 2-63 | Cl | CF₂Cl | -(CH₂)₅- | | |
| 2-64 | Cl | OMe | -(CH₂)₅- | | |
| 2-65 | Cl | NO₂ | -(CH₂)₅- | | |
| 2-66 | Cl | SO₂Me | -(CH₂)₅- | | |
| 2-67 | OMe | Me | -(CH₂)₅- | | |
| 2-68 | OMe | F | -(CH₂)₅- | | |
| 2-69 | OMe | Cl | -(CH₂)₅- | | |
| 2-70 | OMe | Br | -(CH₂)₅- | | |
| 2-71 | OMe | I | -(CH₂)₅- | | |
| 2-72 | OMe | CF₃ | -(CH₂)₅- | | |
| 2-73 | OMe | CHF₂ | -(CH₂)₅- | | |
| 2-74 | OMe | CF₂Cl | -(CH₂)₅- | | |
| 2-75 | OMe | OMe | -(CH₂)₅- | | |
| 2-76 | OMe | NO₂ | -(CH₂)₅- | | |
| 2-77 | OMe | SO₂Me | -(CH₂)₅- | | |
| 2-78 | SO₂Me | Me | -(CH₂)₅- | | |
| 2-79 | SO₂Me | F | -(CH₂)₅- | | |
| 2-80 | SO₂Me | Cl | -(CH₂)₅- | | |
| 2-81 | SO₂Me | Br | -(CH₂)₅- | | |
| 2-82 | SO₂Me | I | -(CH₂)₅- | | |
| 2-83 | SO₂Me | CF₃ | -(CH₂)₅- | | |
| 2-84 | SO₂Me | CHF₂ | -(CH₂)₅- | | |
| 2-85 | SO₂Me | CF₂Cl | -(CH₂)₅- | | |
| 2-86 | SO₂Me | OMe | -(CH₂)₅- | | |
| 2-87 | SO₂Me | NO₂ | -(CH₂)₅- | | |
| 2-88 | SO₂Me | SO₂Me | -(CH₂)₅- | | |
| 2-89 | Me | Me | -(CH₂)₂O(CH₂)₂- | | |
| 2-90 | Me | F | -(CH₂)₂O(CH₂)₂- | | |
| 2-91 | Me | Cl | -(CH₂)₂O(CH₂)₂- | | |
| 2-92 | Me | Br | -(CH₂)₂O(CH₂)₂- | | |
| 2-93 | Me | I | -(CH₂)₂O(CH₂)₂- | | |
| 2-94 | Me | CF₃ | -(CH₂)₂O(CH₂)₂- | | |
| 2-95 | Me | CHF₂ | -(CH₂)₂O(CH₂)₂- | | |
| 2-96 | Me | CF₂Cl | -(CH₂)₂O(CH₂)₂- | | |
| 2-97 | Me | OMe | -(CH₂)₂O(CH₂)₂- | | |
| 2-98 | Me | NO₂ | -(CH₂)₂O(CH₂)₂- | | |
| 2-99 | Me | SO₂Me | -(CH₂)₂O(CH₂)₂- | | |
| 2-100 | Cl | Me | - (CH₂)₂O(CH₂)₂- | | |
| 2-101 | Cl | F | -(CH₂)₂O(CH₂)₂- | | |
| 2-102 | Cl | Cl | -(CH₂)₂O(CH₂)₂- | | (400 MHz, DMSO-d₆ δ, ppm) 7.58 (d,1H), 7.34 (d, 1H), 4.35 (q,2H), 4.20 (m,2H), 4.04 (m,2H), 3.56 - 3.48 (m,2H), 3.48 - 3.41 (m,2H), 1.46 (t,3H) |
| 2-103 | Cl | Br | -(CH₂)₂O(CH₂)₂- | | |
| 2-104 | Cl | I | -(CH₂)₂O(CH₂)₂- | | |
| 2-105 | Cl | CF₃ | -(CH₂)₂O(CH₂)₂- | | |
| 2-106 | Cl | CHF₂ | -(CH₂)₂O(CH₂)₂- | | |
| 2-107 | Cl | CF₂Cl | -(CH₂)₂O(CH₂)₂- | | |
| 2-108 | Cl | OMe | -(CH₂)₂O(CH₂)₂- | | |
| 2-109 | Cl | NO₂ | -(CH₂)₂O(CH₂)₂- | | |
| 2-110 | Cl | SO₂Me | -(CH₂)₂O(CH₂)₂- | | |
| 2-111 | OMe | Me | -(CH₂)₂O(CH₂)₂- | | |
| 2-112 | OMe | F | -(CH₂)₂O(CH₂)₂- | | |
| 2-113 | OMe | Cl | -(CH₂)₂O(CH₂)₂- | | |
| 2-114 | OMe | Br | -(CH₂)₂O(CH₂)₂- | | |
| 2-115 | OMe | I | -(CH₂)₂O(CH₂)₂- | | |
| 2-116 | OMe | CF₃ | -(CH₂)₂O(CH₂)₂- | | |
| 2-117 | OMe | CHF₂ | -(CH₂)₂O(CH₂)₂- | | |
| 2-118 | OMe | CF₂Cl | -(CH₂)₂O(CH₂)₂- | | |
| 2-119 | OMe | OMe | -(CH₂)₂O(CH₂)₂- | | |
| 2-120 | OMe | NO₂ | -(CH₂)₂O(CH₂)₂- | | |
| 2-121 | OMe | SO₂Me | -(CH₂)₂O(CH₂)₂- | | |
| 2-122 | SO₂Me | Me | -(CH₂)₂O(CH₂)₂- | | |
| 2-123 | SO₂Me | F | -(CH₂)₂O(CH₂)₂- | | |
| 2-124 | SO₂Me | Cl | -(CH₂)₂O(CH₂)₂- | | |
| 2-125 | SO₂Me | Br | -(CH₂)₂O(CH₂)₂- | | |
| 2-126 | SO₂Me | I | -(CH₂)₂O(CH₂)₂- | | |
| 2-127 | SO₂Me | CF₃ | -(CH₂)₂O(CH₂)₂- | | |
| 2-128 | SO₂Me | CHF₂ | -(CH₂)₂O(CH₂)₂- | | |
| 2-129 | SO₂Me | CF₂Cl | -(CH₂)₂O(CH₂)₂- | | |
| 2-130 | SO₂Me | OMe | -(CH₂)₂O(CH₂)₂- | | |
| 2-131 | SO₂Me | NO₂ | -(CH₂)₂O(CH₂)₂- | | |
| 2-132 | SO₂Me | SO₂Me | -(CH₂)₂O(CH₂)₂- | | |
| 2-133 | Cl | COOMe | Et | Et | |
| 2-134 | Cl | COOMe | -(CH₂)₅- | | |
| 2-135 | Cl | COOMe | -(CH₂)₂O(CH₂)₂- | | |

**Tabelle 3: Erfindungsgemäße Verbindungen der allgemeinen Formel (I), worin Q für Q1 und R^{x} für eine n-Propylgruppe stehen sowie R" und W jeweils Wasserstoff bedeuten**

| | | | | | |
|---|---|---|---|---|---|
| 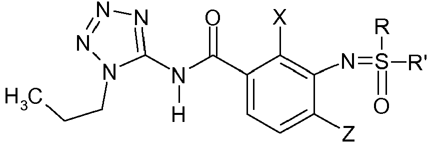 | | | | | |

| Nr. | X | Z | R | R' | Physikalische Daten (¹H-NMR) |
|---|---|---|---|---|---|
| 3-1 | Me | Me | Et | Et | |
| 3-2 | Me | F | Et | Et | |
| 3-3 | Me | Cl | Et | Et | |
| 3-4 | Me | Br | Et | Et | |
| 3-5 | Me | I | Et | Et | |
| 3-6 | Me | CF₃ | Et | Et | |
| 3-7 | Me | CHF₂ | Et | Et | |
| 3-8 | Me | CF₂Cl | Et | Et | |
| 3-9 | Me | OMe | Et | Et | |
| 3-10 | Me | NO₂ | Et | Et | |
| 3-11 | Me | SO₂Me | Et | Et | |
| 3-12 | Cl | Me | Et | Et | |
| 3-13 | Cl | F | Et | Et | |
| 3-14 | Cl | Cl | Et | Et | (400 MHz, DMSO-d₆ δ, ppm) 7.52 (d,1H), 7.25 (d,1H), 4.28 (t,2H), 1.87 (q,2H), 1.33 (t,6H), 0.87 (t,3H) |
| 3-15 | Cl | Br | Et | Et | |
| 3-16 | Cl | I | Et | Et | |
| 3-17 | Cl | CF₃ | Et | Et | |
| 3-18 | Cl | CHF₂ | Et | Et | |
| 3-19 | Cl | CF₂Cl | Et | Et | |
| 3-20 | Cl | OMe | Et | Et | |
| 3-21 | Cl | NO₂ | Et | Et | |
| 3-22 | Cl | SO₂Me | Et | Et | |
| 3-23 | OMe | Me | Et | Et | |
| 3-24 | OMe | F | Et | Et | |
| 3-25 | OMe | Cl | Et | Et | |
| 3-26 | OMe | Br | Et | Et | |
| 3-27 | OMe | I | Et | Et | |
| 3-28 | OMe | CF₃ | Et | Et | |
| 3-29 | OMe | CHF₂ | Et | Et | |
| 3-30 | OMe | CF₂Cl | Et | Et | |
| 3-31 | OMe | OMe | Et | Et | |
| 3-32 | OMe | NO₂ | Et | Et | |
| 3-33 | OMe | SO₂Me | Et | Et | |
| 3-34 | SO₂Me | Me | Et | Et | |
| 3-35 | SO₂Me | F | Et | Et | |
| 3-36 | SO₂Me | Cl | Et | Et | |
| 3-37 | SO₂Me | Br | Et | Et | |
| 3-38 | SO₂Me | I | Et | Et | |
| 3-39 | SO₂Me | CF₃ | Et | Et | |
| 3-40 | SO₂Me | CHF₂ | Et | Et | |
| 3-41 | SO₂Me | CF₂Cl | Et | Et | |
| 3-42 | SO₂Me | OMe | Et | Et | |
| 3-43 | SO₂Me | NO₂ | Et | Et | |
| 3-44 | SO₂Me | SO₂Me | Et | Et | |
| 3-45 | Me | Me | -(CH₂)₅- | | |
| 3-46 | Me | F | -(CH₂)₅- | | |
| 3-47 | Me | Cl | -(CH₂)₅- | | |
| 3-48 | Me | Br | -(CH₂)₅- | | |
| 3-49 | Me | I | -(CH₂)₅- | | |
| 3-50 | Me | CF₃ | -(CH₂)₅- | | |
| 3-51 | Me | CHF₂ | -(CH₂)₅- | | |
| 3-52 | Me | CF₂Cl | -(CH₂)₅- | | |
| 3-53 | Me | OMe | -(CH₂)₅- | | |
| 3-54 | Me | NO₂ | -(CH₂)₅- | | |
| 3-55 | Me | SO₂Me | -(CH₂)₅- | | |
| 3-56 | Cl | Me | -(CH₂)₅- | | |
| 3-57 | Cl | F | -(CH₂)₅- | | |
| 3-58 | Cl | Cl | -(CH₂)₅- | | (400 MHz, DMSO-d₆ δ, ppm) 7.54 (d, 1H), 7.27 (d,1H), 4.29 (t,2H), 3.42 - 3.25 (m,4H), 2.13 - 2.05 (m,2H), 2.00 - 1.82 (m,4H), 1.70 - 1.51 (m,2H), 0.87 (t,3H) |
| 3-59 | Cl | Br | -(CH₂)₅- | | |
| 3-60 | Cl | I | -(CH₂)₅- | | |
| 3-61 | Cl | CF₃ | -(CH₂)₅- | | |
| 3-62 | Cl | CHF₂ | -(CH₂)₅- | | |
| 3-63 | Cl | CF₂Cl | -(CH₂)₅- | | |
| 3-64 | Cl | OMe | -(CH₂)₅- | | |
| 3-65 | Cl | NO₂ | -(CH₂)₅- | | |
| 3-66 | Cl | SO₂Me | -(CH₂)₅- | | |
| 3-67 | OMe | Me | -(CH₂)₅- | | |
| 3-68 | OMe | F | -(CH₂)₅- | | |
| 3-69 | OMe | Cl | -(CH₂)₅- | | |
| 3-70 | OMe | Br | -(CH₂)₅- | | |
| 3-71 | OMe | I | -(CH₂)₅- | | |
| 3-72 | OMe | CF₃ | -(CH₂)₅- | | |
| 3-73 | OMe | CHF₂ | -(CH₂)₅- | | |
| 3-74 | OMe | CF₂Cl | -(CH₂)₅- | | |
| 3-75 | OMe | OMe | -(CH₂)₅- | | |
| 3-76 | OMe | NO₂ | -(CH₂)₅- | | |
| 3-77 | OMe | SO₂Me | -(CH₂)₅- | | |
| 3-78 | SO₂Me | Me | -(CH₂)₅- | | |
| 3-79 | SO₂Me | F | -(CH₂)₅- | | |
| 3-80 | SO₂Me | Cl | -(CH₂)₅- | | |
| 3-81 | SO₂Me | Br | -(CH₂)₅- | | |
| 3-82 | SO₂Me | I | -(CH₂)₅- | | |
| 3-83 | SO₂Me | CF₃ | -(CH₂)₅- | | |
| 3-84 | SO₂Me | CHF₂ | -(CH₂)₅- | | |
| 3-85 | SO₂Me | CF₂Cl | -(CH₂)₅- | | |
| 3-86 | SO₂Me | OMe | -(CH₂)₅- | | |
| 3-87 | SO₂Me | NO₂ | -(CH₂)₅- | | |
| 3-88 | SO₂Me | SO₂Me | -(CH₂)₅- | | |
| 3-89 | Me | Me | -(CH₂)₂O(CH₂)₂- | | |
| 3-90 | Me | F | -(CH₂)₂O(CH₂)₂- | | |
| 3-91 | Me | Cl | -(CH₂)₂O(CH₂)₂- | | |
| 3-92 | Me | Br | -(CH₂)₂O(CH₂)₂- | | |
| 3-93 | Me | I | -(CH₂)₂O(CH₂)₂- | | |
| 3-94 | Me | CF₃ | -(CH₂)₂O(CH₂)₂- | | |
| 3-95 | Me | CHF₂ | -(CH₂)₂O(CH₂)₂- | | |
| 3-96 | Me | CF₂Cl | -(CH₂)₂O(CH₂)₂- | | |
| 3-97 | Me | OMe | -(CH₂)₂O(CH₂)₂- | | |
| 3-98 | Me | NO₂ | -(CH₂)₂O(CH₂)₂- | | |
| 3-99 | Me | SO₂Me | -(CH₂)₂O(CH₂)₂- | | |
| 3-100 | Cl | Me | -(CH₂)₂O(CH₂)₂- | | |
| 3-101 | Cl | F | -(CH₂)₂O(CH₂)₂- | | |
| 3-102 | Cl | Cl | -(CH₂)₂O(CH₂)₂- | | (400 MHz, DMSO-d₆ δ, ppm) 7.57 (d,1H), 7.31 (d,1H), 4.29 (t,2H), 4.22 - 4.15 (m,2H), 4.02 (m,2H), 3.55 - 3.40 (m,4H), 1.87 (m,2H), 0.87 (t,3H) |
| 3-103 | Cl | Br | -(CH₂)₂O(CH₂)₂- | | |
| 3-104 | Cl | I | -(CH₂)₂O(CH₂)₂- | | |
| 3-105 | Cl | CF₃ | -(CH₂)₂O(CH₂)₂- | | |
| 3-106 | Cl | CHF₂ | -(CH₂)₂O(CH₂)₂- | | |
| 3-107 | Cl | CF₂Cl | -(CH₂)₂O(CH₂)₂- | | |
| 3-108 | Cl | OMe | -(CH₂)₂O(CH₂)₂- | | |
| 3-109 | Cl | NO₂ | -(CH₂)₂O(CH₂)₂- | | |
| 3-110 | Cl | SO₂Me | -(CH₂)₂O(CH₂)₂- | | |
| 3-111 | OMe | Me | -(CH₂)₂O(CH₂)₂- | | |
| 3-112 | OMe | F | -(CH₂)₂O(CH₂)₂- | | |
| 3-113 | OMe | Cl | -(CH₂)₂O(CH₂)₂- | | |
| 3-114 | OMe | Br | -(CH₂)₂O(CH₂)₂- | | |
| 3-115 | OMe | I | -(CH₂)₂O(CH₂)₂- | | |
| 3-116 | OMe | CF₃ | -(CH₂)₂O(CH₂)₂- | | |
| 3-117 | OMe | CHF₂ | -(CH₂)₂O(CH₂)₂- | | |
| 3-118 | OMe | CF₂Cl | -(CH₂)₂O(CH₂)₂- | | |
| 3-119 | OMe | OMe | -(CH₂)₂O(CH₂)₂- | | |
| 3-120 | OMe | NO₂ | -(CH₂)₂O(CH₂)₂- | | |
| 3-121 | OMe | SO₂Me | -(CH₂)₂O(CH₂)₂- | | |
| 3-122 | SO₂Me | Me | -(CH₂)₂O(CH₂)₂- | | |
| 3-123 | SO₂Me | F | -(CH₂)₂O(CH₂)₂- | | |
| 3-124 | SO₂Me | Cl | -(CH₂)₂O(CH₂)₂- | | |
| 3-125 | SO₂Me | Br | -(CH₂)₂O(CH₂)₂- | | |
| 3-126 | SO₂Me | I | -(CH₂)₂O(CH₂)₂- | | |
| 3-127 | SO₂Me | CF₃ | -(CH₂)₂O(CH₂)₂- | | |
| 3-128 | SO₂Me | CHF₂ | -(CH₂)₂O(CH₂)₂- | | |
| 3-129 | SO₂Me | CF₂Cl | -(CH₂)₂O(CH₂)₂- | | |
| 3-130 | SO₂Me | OMe | -(CH₂)₂O(CH₂)₂- | | |
| 3-131 | SO₂Me | NO₂ | -(CH₂)₂O(CH₂)₂- | | |
| 3-132 | SO₂Me | SO₂Me | -(CH₂)₂O(CH₂)₂- | | |
| 3-133 | Cl | COOMe | Et | Et | |
| 3-134 | Cl | COOMe | -(CH₂)₅- | | |
| 3-135 | Cl | COOMe | -(CH₂)₂O(CH₂)₂- | | |

**Tabelle 4: Erfindungsgemäße Verbindungen der allgemeinen Formel (I), worin Q für Q1 und R^{x} für eine (2-Methoxyethyl)gruppe stehen sowie R" und W jeweils Wasserstoff bedeuten**

| | | | | | |
|---|---|---|---|---|---|
| 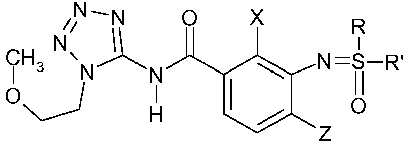 | | | | | |

| Nr. | X | Z | R | R' | Physikalische Daten (¹H-NMR) |
|---|---|---|---|---|---|
| 4-1 | Me | Me | Et | Et | |
| 4-2 | Me | F | Et | Et | |
| 4-3 | Me | Cl | Et | Et | |
| 4-4 | Me | Br | Et | Et | |
| 4-5 | Me | I | Et | Et | |
| 4-6 | Me | CF₃ | Et | Et | |
| 4-7 | Me | CHF₂ | Et | Et | |
| 4-8 | Me | CF₂Cl | Et | Et | |
| 4-9 | Me | OMe | Et | Et | |
| 4-10 | Me | NO₂ | Et | Et | |
| 4-11 | Me | SO₂Me | Et | Et | |
| 4-12 | Cl | Me | Et | Et | |
| 4-13 | Cl | F | Et | Et | |
| 4-14 | Cl | Cl | Et | Et | |
| 4-15 | Cl | Br | Et | Et | |
| 4-16 | Cl | I | Et | Et | |
| 4-17 | Cl | CF₃ | Et | Et | |
| 4-18 | Cl | CHF₂ | Et | Et | |
| 4-19 | Cl | CF₂Cl | Et | Et | |
| 4-20 | Cl | OMe | Et | Et | |
| 4-21 | Cl | NO₂ | Et | Et | |
| 4-22 | Cl | SO₂Me | Et | Et | |
| 4-23 | OMe | Me | Et | Et | |
| 4-24 | OMe | F | Et | Et | |
| 4-25 | OMe | Cl | Et | Et | |
| 4-26 | OMe | Br | Et | Et | |
| 4-27 | OMe | I | Et | Et | |
| 4-28 | OMe | CF₃ | Et | Et | |
| 4-29 | OMe | CHF₂ | Et | Et | |
| 4-30 | OMe | CF₂Cl | Et | Et | |
| 4-31 | OMe | OMe | Et | Et | |
| 4-32 | OMe | NO₂ | Et | Et | |
| 4-33 | OMe | SO₂Me | Et | Et | |
| 4-34 | SO₂Me | Me | Et | Et | |
| 4-35 | SO₂Me | F | Et | Et | |
| 4-36 | SO₂Me | Cl | Et | Et | |
| 4-37 | SO₂Me | Br | Et | Et | |
| 4-38 | SO₂Me | I | Et | Et | |
| 4-39 | SO₂Me | CF₃ | Et | Et | |
| 4-40 | SO₂Me | CHF₂ | Et | Et | |
| 4-41 | SO₂Me | CF₂Cl | Et | Et | |
| 4-42 | SO₂Me | OMe | Et | Et | |
| 4-43 | SO₂Me | NO₂ | Et | Et | |
| 4-44 | SO₂Me | SO₂Me | Et | Et | |
| 4-45 | Me | Me | -(CH₂)₅- | | |
| 4-46 | Me | F | -(CH₂)₅- | | |
| 4-47 | Me | Cl | -(CH₂)₅- | | |
| 4-48 | Me | Br | -(CH₂)₅- | | |
| 4-49 | Me | I | -(CH₂)₅- | | |
| 4-50 | Me | CF₃ | -(CH₂)₅- | | |
| 4-51 | Me | CHF₂ | -(CH₂)₅- | | |
| 4-52 | Me | CF₂Cl | -(CH₂)₅- | | |
| 4-53 | Me | OMe | -(CH₂)₅- | | |
| 4-54 | Me | NO₂ | -(CH₂)₅- | | |
| 4-55 | Me | SO₂Me | -(CH₂)₅- | | |
| 4-56 | Cl | Me | -(CH₂)₅- | | |
| 4-57 | Cl | F | -(CH₂)₅- | | |
| 4-58 | Cl | Cl | -(CH₂)₅- | | |
| 4-59 | Cl | Br | -(CH₂)₅- | | |
| 4-60 | Cl | I | -(CH₂)₅- | | |
| 4-61 | Cl | CF₃ | -(CH₂)₅- | | |
| 4-62 | Cl | CHF₂ | -(CH₂)₅- | | |
| 4-63 | Cl | CF₂Cl | -(CH₂)₅- | | |
| 4-64 | Cl | OMe | -(CH₂)₅- | | |
| 4-65 | Cl | NO₂ | -(CH₂)₅- | | |
| 4-66 | Cl | SO₂Me | -(CH₂)₅- | | |
| 4-67 | OMe | Me | -(CH₂)₅- | | |
| 4-68 | OMe | F | -(CH₂)₅- | | |
| 4-69 | OMe | Cl | -(CH₂)₅- | | |
| 4-70 | OMe | Br | -(CH₂)₅- | | |
| 4-71 | OMe | I | -(CH₂)₅- | | |
| 4-72 | OMe | CF₃ | -(CH₂)₅- | | |
| 4-73 | OMe | CHF₂ | -(CH₂)₅- | | |
| 4-74 | OMe | CF₂Cl | -(CH₂)₅- | | |
| 4-75 | OMe | OMe | -(CH₂)₅- | | |
| 4-76 | OMe | NO₂ | -(CH₂)₅- | | |
| 4-77 | OMe | SO₂Me | -(CH₂)₅- | | |
| 4-78 | SO₂Me | Me | -(CH₂)₅- | | |
| 4-79 | SO₂Me | F | -(CH₂)₅- | | |
| 4-80 | SO₂Me | Cl | -(CH₂)₅- | | |
| 4-81 | SO₂Me | Br | -(CH₂)₅- | | |
| 4-82 | SO₂Me | I | -(CH₂)₅- | | |
| 4-83 | SO₂Me | CF₃ | -(CH₂)₅- | | |
| 4-84 | SO₂Me | CHF₂ | -(CH₂)₅- | | |
| 4-85 | SO₂Me | CF₂Cl | -(CH₂)₅- | | |
| 4-86 | SO₂Me | OMe | -(CH₂)₅- | | |
| 4-87 | SO₂Me | NO₂ | -(CH₂)₅- | | |
| 4-88 | SO₂Me | SO₂Me | -(CH₂)₅- | | |
| 4-89 | Me | Me | -(CH₂)₂O(CH₂)₂- | | |
| 4-90 | Me | F | -(CH₂)₂O(CH₂)₂- | | |
| 4-91 | Me | Cl | -(CH₂)₂O(CH₂)₂- | | |
| 4-92 | Me | Br | -(CH₂)₂O(CH₂)₂- | | |
| 4-93 | Me | I | -(CH₂)₂O(CH₂)₂- | | |
| 4-94 | Me | CF₃ | -(CH₂)₂O(CH₂)₂- | | |
| 4-95 | Me | CHF₂ | -(CH₂)₂O(CH₂)₂- | | |
| 4-96 | Me | CF₂Cl | -(CH₂)₂O(CH₂)₂- | | |
| 4-97 | Me | OMe | -(CH₂)₂O(CH₂)₂- | | |
| 4-98 | Me | NO₂ | -(CH₂)₂O(CH₂)₂- | | |
| 4-99 | Me | SO₂Me | -(CH₂)₂O(CH₂)₂- | | |
| 4-100 | Cl | Me | -(CH₂)₂O(CH₂)₂- | | |
| 4-101 | Cl | F | -(CH₂)₂O(CH₂)₂- | | |
| 4-102 | Cl | Cl | -(CH₂)₂O(CH₂)₂- | | |
| 4-103 | Cl | Br | -(CH₂)₂O(CH₂)₂- | | |
| 4-104 | Cl | I | -(CH₂)₂O(CH₂)₂- | | |
| 4-105 | Cl | CF₃ | -(CH₂)₂O(CH₂)₂- | | |
| 4-106 | Cl | CHF₂ | -(CH₂)₂O(CH₂)₂- | | |
| 4-107 | Cl | CF₂Cl | -(CH₂)₂O(CH₂)₂- | | |
| 4-108 | Cl | OMe | -(CH₂)₂O(CH₂)₂- | | |
| 4-109 | Cl | NO₂ | -(CH₂)₂O(CH₂)₂- | | |
| 4-110 | Cl | SO₂Me | -(CH₂)₂O(CH₂)₂- | | |
| 4-111 | OMe | Me | -(CH₂)₂O(CH₂)₂- | | |
| 4-112 | OMe | F | -(CH₂)₂O(CH₂)₂- | | |
| 4-113 | OMe | Cl | -(CH₂)₂O(CH₂)₂- | | |
| 4-114 | OMe | Br | -(CH₂)₂O(CH₂)₂- | | |
| 4-115 | OMe | I | -(CH₂)₂O(CH₂)₂- | | |
| 4-116 | OMe | CF₃ | -(CH₂)₂O(CH₂)₂- | | |
| 4-117 | OMe | CHF₂ | -(CH₂)₂O(CH₂)₂- | | |
| 4-118 | OMe | CF₂Cl | -(CH₂)₂O(CH₂)₂- | | |
| 4-119 | OMe | OMe | -(CH₂)₂O(CH₂)₂- | | |
| 4-120 | OMe | NO₂ | -(CH₂)₂O(CH₂)₂- | | |
| 4-121 | OMe | SO₂Me | -(CH₂)₂O(CH₂)₂- | | |
| 4-122 | SO₂Me | Me | -(CH₂)₂O(CH₂)₂- | | |
| 4-123 | SO₂Me | F | -(CH₂)₂O(CH₂)₂- | | |
| 4-124 | SO₂Me | Cl | -(CH₂)₂O(CH₂)₂- | | |
| 4-125 | SO₂Me | Br | -(CH₂)₂O(CH₂)₂- | | |
| 4-126 | SO₂Me | I | -(CH₂)₂O(CH₂)₂- | | |
| 4-127 | SO₂Me | CF₃ | -(CH₂)₂O(CH₂)₂- | | |
| 4-128 | SO₂Me | CHF₂ | -(CH₂)₂O(CH₂)₂- | | |
| 4-129 | SO₂Me | CF₂Cl | -(CH₂)₂O(CH₂)₂- | | |
| 4-130 | SO₂Me | OMe | -(CH₂)₂O(CH₂)₂- | | |
| 4-131 | SO₂Me | NO₂ | -(CH₂)₂O(CH₂)₂- | | |
| 4-132 | SO₂Me | SO₂Me | -(CH₂)₂O(CH₂)₂- | | |
| 4-133 | Cl | COOMe | Et | Et | |
| 4-134 | Cl | COOMe | -(CH₂)₅- | | |
| 4-135 | Cl | COOMe | -(CH₂)₂O(CH₂)₂- | | |

**Tabelle 5: Erfindungsgemäße Verbindungen der allgemeinen Formel (I), worin Q für Q2 und R^{x} für eine Methylgruppe stehen sowie R" und W jeweils Wasserstoff bedeuten**

| | | | | | |
|---|---|---|---|---|---|
| 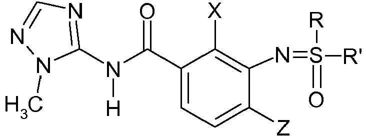 | | | | | |

| Nr. | X | Z | R | R' | Physikalische Daten (¹H-NMR) |
|---|---|---|---|---|---|
| 5-1 | Me | Me | Et | Et | |
| 5-2 | Me | F | Et | Et | |
| 5-3 | Me | Cl | Et | Et | |
| 5-4 | Me | Br | Et | Et | |
| 5-5 | Me | I | Et | Et | |
| 5-6 | Me | CF₃ | Et | Et | |
| 5-7 | Me | CHF₂ | Et | Et | |
| 5-8 | Me | CF₂Cl | Et | Et | |
| 5-9 | Me | OMe | Et | Et | |
| 5-10 | Me | NO₂ | Et | Et | |
| 5-11 | Me | SO₂Me | Et | Et | |
| 5-12 | Cl | Me | Et | Et | |
| 5-13 | Cl | F | Et | Et | |
| 5-14 | Cl | Cl | Et | Et | |
| 5-15 | Cl | Br | Et | Et | |
| 5-16 | Cl | I | Et | Et | |
| 5-17 | Cl | CF₃ | Et | Et | |
| 5-18 | Cl | CHF₂ | Et | Et | |
| 5-19 | Cl | CF₂Cl | Et | Et | |
| 5-20 | Cl | OMe | Et | Et | |
| 5-21 | Cl | NO₂ | Et | Et | |
| 5-22 | Cl | SO₂Me | Et | Et | |
| 5-23 | OMe | Me | Et | Et | |
| 5-24 | OMe | F | Et | Et | |
| 5-25 | OMe | Cl | Et | Et | |
| 5-26 | OMe | Br | Et | Et | |
| 5-27 | OMe | I | Et | Et | |
| 5-28 | OMe | CF₃ | Et | Et | |
| 5-29 | OMe | CHF₂ | Et | Et | |
| 5-30 | OMe | CF₂Cl | Et | Et | |
| 5-31 | OMe | OMe | Et | Et | |
| 5-32 | OMe | NO₂ | Et | Et | |
| 5-33 | OMe | SO₂Me | Et | Et | |
| 5-34 | SO₂Me | Me | Et | Et | |
| 5-35 | SO₂Me | F | Et | Et | |
| 5-36 | SO₂Me | Cl | Et | Et | |
| 5-37 | SO₂Me | Br | Et | Et | |
| 5-38 | SO₂Me | I | Et | Et | |
| 5-39 | SO₂Me | CF₃ | Et | Et | |
| 5-40 | SO₂Me | CHF₂ | Et | Et | |
| 5-41 | SO₂Me | CF₂Cl | Et | Et | |
| 5-42 | SO₂Me | OMe | Et | Et | |
| 5-43 | SO₂Me | NO₂ | Et | Et | |
| 5-44 | SO₂Me | SO₂Me | Et | Et | |
| 5-45 | Me | Me | - (CH₂)₅ - | | |
| 5-46 | Me | F | - (CH₂)₅ - | | |
| 5-47 | Me | Cl | - (CH₂)₅ - | | |
| 5-48 | Me | Br | - (CH₂)₅ - | | |
| 5-49 | Me | I | - (CH₂)₅ - | | |
| 5-50 | Me | CF₃ | - (CH₂)₅ - | | |
| 5-51 | Me | CHF₂ | - (CH₂)₅ - | | |
| 5-52 | Me | CF₂Cl | - (CH₂)₅ - | | |
| 5-53 | Me | OMe | - (CH₂)₅ - | | |
| 5-54 | Me | NO₂ | - (CH₂)₅ - | | |
| 5-55 | Me | SO₂Me | - (CH₂)₅ - | | |
| 5-56 | Cl | Me | - (CH₂)₅ - | | |
| 5-57 | Cl | F | - (CH₂)₅ - | | |
| 5-58 | Cl | Cl | - (CH₂)₅ - | | |
| 5-59 | Cl | Br | - (CH₂)₅ - | | |
| 5-60 | Cl | I | - (CH₂)₅ - | | |
| 5-61 | Cl | CF₃ | - (CH₂)₅ - | | |
| 5-62 | Cl | CHF₂ | - (CH₂)₅ - | | |
| 5-63 | Cl | CF₂Cl | - (CH₂)₅ - | | |
| 5-64 | Cl | OMe | - (CH₂)₅ - | | |
| 5-65 | Cl | NO₂ | - (CH₂)₅ - | | |
| 5-66 | Cl | SO₂Me | - (CH₂)₅ - | | |
| 5-67 | OMe | Me | - (CH₂)₅ - | | |
| 5-68 | OMe | F | - (CH₂)₅ - | | |
| 5-69 | OMe | Cl | - (CH₂)₅ - | | |
| 5-70 | OMe | Br | - (CH₂)₅ - | | |
| 5-71 | OMe | I | - (CH₂)₅ - | | |
| 5-72 | OMe | CF₃ | - (CH₂)₅ - | | |
| 5-73 | OMe | CHF₂ | - (CH₂)₅ - | | |
| 5-74 | OMe | CF₂Cl | - (CH₂)₅ - | | |
| 5-75 | OMe | OMe | - (CH₂)₅ - | | |
| 5-76 | OMe | NO₂ | - (CH₂)₅ - | | |
| 5-77 | OMe | SO₂Me | - (CH₂)₅ - | | |
| 5-78 | SO₂Me | Me | - (CH₂)₅ - | | |
| 5-79 | SO₂Me | F | - (CH₂)₅ - | | |
| 5-80 | SO₂Me | Cl | - (CH₂)₅ - | | |
| 5-81 | SO₂Me | Br | - (CH₂)₅ - | | |
| 5-82 | SO₂Me | I | - (CH₂)₅ - | | |
| 5-83 | SO₂Me | CF₃ | - (CH₂)₅ - | | |
| 5-84 | SO₂Me | CHF₂ | - (CH₂)₅ - | | |
| 5-85 | SO₂Me | CF₂Cl | - (CH₂)₅ - | | |
| 5-86 | SO₂Me | OMe | - (CH₂)₅ - | | |
| 5-87 | SO₂Me | NO₂ | - (CH₂)₅ - | | |
| 5-88 | SO₂Me | SO₂Me | - (CH₂)₅ - | | |
| 5-89 | Me | Me | - (CH₂)₂O(CH₂)₂ - | | |
| 5-90 | Me | F | - (CH₂)₂O(CH₂)₂ - | | |
| 5-91 | Me | Cl | - (CH₂)₂O(CH₂)₂ - | | |
| 5-92 | Me | Br | - (CH₂)₂O(CH₂)₂ - | | |
| 5-93 | Me | I | - (CH₂)₂O(CH₂)₂ - | | |
| 5-94 | Me | CF₃ | - (CH₂)₂O(CH₂)₂ - | | |
| 5-95 | Me | CHF₂ | - (CH₂)₂O(CH₂)₂ - | | |
| 5-96 | Me | CF₂Cl | - (CH₂)₂O(CH₂)₂ - | | |
| 5-97 | Me | OMe | - (CH₂)₂O(CH₂)₂ - | | |
| 5-98 | Me | NO₂ | - (CH₂)₂O(CH₂)₂ - | | |
| 5-99 | Me | SO₂Me | - (CH₂)₂O(CH₂)₂ - | | |
| 5-100 | Cl | Me | - (CH₂)₂O(CH₂)₂ - | | |
| 5-101 | Cl | F | - (CH₂)₂O(CH₂)₂ - | | |
| 5-102 | Cl | Cl | - (CH₂)₂O(CH₂)₂ - | | |
| 5-103 | Cl | Br | - (CH₂)₂O(CH₂)₂ - | | |
| 5-104 | Cl | I | - (CH₂)₂O(CH₂)₂ - | | |
| 5-105 | Cl | CF₃ | - (CH₂)₂O(CH₂)₂ - | | |
| 5-106 | Cl | CHF₂ | - (CH₂)₂O(CH₂)₂ - | | |
| 5-107 | Cl | CF₂Cl | - (CH₂)₂O(CH₂)₂ - | | |
| 5-108 | Cl | OMe | - (CH₂)₂O(CH₂)₂ - | | |
| 5-109 | Cl | NO₂ | - (CH₂)₂O(CH₂)₂ - | | |
| 5-110 | Cl | SO₂Me | - (CH₂)₂O(CH₂)₂ - | | |
| 5-111 | OMe | Me | - (CH₂)₂O(CH₂)₂ - | | |
| 5-112 | OMe | F | - (CH₂)₂O(CH₂)₂ - | | |
| 5-113 | OMe | Cl | - (CH₂)₂O(CH₂)₂ - | | |
| 5-114 | OMe | Br | - (CH₂)₂O(CH₂)₂ - | | |
| 5-115 | OMe | I | - (CH₂)₂O(CH₂)₂ - | | |
| 5-116 | OMe | CF₃ | - (CH₂)₂O(CH₂)₂ - | | |
| 5-117 | OMe | CHF₂ | - (CH₂)₂O(CH₂)₂ - | | |
| 5-118 | OMe | CF₂Cl | - (CH₂)₂O(CH₂)₂ - | | |
| 5-119 | OMe | OMe | - (CH₂)₂O(CH₂)₂ - | | |
| 5-120 | OMe | NO₂ | - (CH₂)₂O(CH₂)₂ - | | |
| 5-121 | OMe | SO₂Me | - (CH₂)₂O(CH₂)₂ - | | |
| 5-122 | SO₂Me | Me | - (CH₂)₂O(CH₂)₂ - | | |
| 5-123 | SO₂Me | F | - (CH₂)₂O(CH₂)₂ - | | |
| 5-124 | SO₂Me | Cl | - (CH₂)₂O(CH₂)₂ - | | |
| 5-125 | SO₂Me | Br | - (CH₂)₂O(CH₂)₂ - | | |
| 5-126 | SO₂Me | I | - (CH₂)₂O(CH₂)₂ - | | |
| 5-127 | SO₂Me | CF₃ | - (CH₂)₂O(CH₂)₂ - | | |
| 5-128 | SO₂Me | CHF₂ | - (CH₂)₂O(CH₂)₂ - | | |
| 5-129 | SO₂Me | CF₂Cl | - (CH₂)₂O(CH₂)₂ - | | |
| 5-130 | SO₂Me | OMe | - (CH₂)₂O(CH₂)₂ - | | |
| 5-131 | SO₂Me | NO₂ | - (CH₂)₂O(CH₂)₂ - | | |
| 5-132 | SO₂Me | SO₂Me | - (CH₂)₂O(CH₂)₂ - | | |
| 5-133 | Cl | COOMe | Et | Et | |
| 5-134 | Cl | COOMe | - (CH₂)₅ - | | |
| 5-135 | Cl | COOMe | - (CH₂)₂O(CH₂)₂ - | | |

**Tabelle 6: Erfindungsgemäße Verbindungen der allgemeinen Formel (I), worin Q für Q3 und R^{y} für eine Methylgruppe stehen sowie R" und W jeweils Wasserstoff bedeuten**

| | | | | | |
|---|---|---|---|---|---|
| 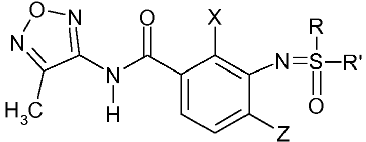 | | | | | |

| Nr. | X | Z | R | R' | Physikalische Daten (¹H-NMR) |
|---|---|---|---|---|---|
| 6-1 | Me | Me | Et | Et | |
| 6-2 | Me | F | Et | Et | |
| 6-3 | Me | Cl | Et | Et | |
| 6-4 | Me | Br | Et | Et | |
| 6-5 | Me | I | Et | Et | |
| 6-6 | Me | CF₃ | Et | Et | |
| 6-7 | Me | CHF₂ | Et | Et | |
| 6-8 | Me | CF₂Cl | Et | Et | |
| 6-9 | Me | OMe | Et | Et | |
| 6-10 | Me | NO₂ | Et | Et | |
| 6-11 | Me | SO₂Me | Et | Et | |
| 6-12 | Cl | Me | Et | Et | |
| 6-13 | Cl | F | Et | Et | |
| 6-14 | Cl | Cl | Et | Et | (400 MHz, CDCl₃ δ, ppm) 7.37 (d,1H), 7.25 (d,1H), 3.30 (q,4H), 2.48 (s,3H), 1.47 (t,6H) |
| 6-15 | Cl | Br | Et | Et | |
| 6-16 | Cl | I | Et | Et | |
| 6-17 | Cl | CF₃ | Et | Et | |
| 6-18 | Cl | CHF₂ | Et | Et | |
| 6-19 | Cl | CF₂Cl | Et | Et | |
| 6-20 | Cl | OMe | Et | Et | |
| 6-21 | Cl | NO₂ | Et | Et | |
| 6-22 | Cl | SO₂Me | Et | Et | |
| 6-23 | OMe | Me | Et | Et | |
| 6-24 | OMe | F | Et | Et | |
| 6-25 | OMe | Cl | Et | Et | |
| 6-26 | OMe | Br | Et | Et | |
| 6-27 | OMe | I | Et | Et | |
| 6-28 | OMe | CF₃ | Et | Et | |
| 6-29 | OMe | CHF₂ | Et | Et | |
| 6-30 | OMe | CF₂Cl | Et | Et | |
| 6-31 | OMe | OMe | Et | Et | |
| 6-32 | OMe | NO₂ | Et | Et | |
| 6-33 | OMe | SO₂Me | Et | Et | |
| 6-34 | SO₂Me | Me | Et | Et | |
| 6-35 | SO₂Me | F | Et | Et | |
| 6-36 | SO₂Me | Cl | Et | Et | |
| 6-37 | SO₂Me | Br | Et | Et | |
| 6-38 | SO₂Me | I | Et | Et | |
| 6-39 | SO₂Me | CF₃ | Et | Et | |
| 6-40 | SO₂Me | CHF₂ | Et | Et | |
| 6-41 | SO₂Me | CF₂Cl | Et | Et | |
| 6-42 | SO₂Me | OMe | Et | Et | |
| 6-43 | SO₂Me | NO₂ | Et | Et | |
| 6-44 | SO₂Me | SO₂Me | Et | Et | |
| 6-45 | Me | Me | - (CH₂)₅ - | | |
| 6-46 | Me | F | - (CH₂)₅ - | | |
| 6-47 | Me | Cl | - (CH₂)₅ - | | |
| 6-48 | Me | Br | - (CH₂)₅ - | | |
| 6-49 | Me | I | - (CH₂)₅ - | | |
| 6-50 | Me | CF₃ | - (CH₂)₅ - | | |
| 6-51 | Me | CHF₂ | - (CH₂)₅ - | | |
| 6-52 | Me | CF₂Cl | - (CH₂)₅ - | | |
| 6-53 | Me | OMe | - (CH₂)₅ - | | |
| 6-54 | Me | NO₂ | - (CH₂)₅ - | | |
| 6-55 | Me | SO₂Me | - (CH₂)₅ - | | |
| 6-56 | Cl | Me | - (CH₂)₅ - | | |
| 6-57 | Cl | F | - (CH₂)₅ - | | |
| 6-58 | Cl | Cl | - (CH₂)₅ - | | (400 MHz, DMSO-d₆ δ, ppm) 7.54 (d,1H), 7.26 (d,1H), 3.43 - 3.26 (m,4H), 2.38 (s,3H), 2.13 - 2.05 (m,2H), 1.94 (m,2H), 1.62 (m,2H) |
| 6-59 | Cl | Br | - (CH₂)₅ - | | |
| 6-60 | Cl | I | - (CH₂)₅ - | | |
| 6-61 | Cl | CF₃ | - (CH₂)₅ - | | |
| 6-62 | Cl | CHF₂ | - (CH₂)₅ - | | |
| 6-63 | Cl | CF₂Cl | - (CH₂)₅ - | | |
| 6-64 | Cl | OMe | - (CH₂)₅ - | | |
| 6-65 | Cl | NO₂ | - (CH₂)₅ - | | |
| 6-66 | Cl | SO₂Me | - (CH₂)₅ - | | |
| 6-67 | OMe | Me | - (CH₂)₅ - | | |
| 6-68 | OMe | F | - (CH₂)₅ - | | |
| 6-69 | OMe | Cl | - (CH₂)₅ - | | |
| 6-70 | OMe | Br | - (CH₂)₅ - | | |
| 6-71 | OMe | I | - (CH₂)₅ - | | |
| 6-72 | OMe | CF₃ | - (CH₂)₅ - | | |
| 6-73 | OMe | CHF₂ | - (CH₂)₅ - | | |
| 6-74 | OMe | CF₂Cl | - (CH₂)₅ - | | |
| 6-75 | OMe | OMe | - (CH₂)₅ - | | |
| 6-76 | OMe | NO₂ | - (CH₂)₅ - | | |
| 6-77 | OMe | SO₂Me | - (CH₂)₅ - | | |
| 6-78 | SO₂Me | Me | - (CH₂)₅ - | | |
| 6-79 | SO₂Me | F | - (CH₂)₅ - | | |
| 6-80 | SO₂Me | Cl | - (CH₂)₅ - | | |
| 6-81 | SO₂Me | Br | - (CH₂)₅ - | | |
| 6-82 | SO₂Me | I | - (CH₂)₅ - | | |
| 6-83 | SO₂Me | CF₃ | - (CH₂)₅ - | | |
| 6-84 | SO₂Me | CHF₂ | - (CH₂)₅ - | | |
| 6-85 | SO₂Me | CF₂Cl | - (CH₂)₅ - | | |
| 6-86 | SO₂Me | OMe | - (CH₂)₅ - | | |
| 6-87 | SO₂Me | NO₂ | - (CH₂)₅ - | | |
| 6-88 | SO₂Me | SO₂Me | - (CH₂)₅ - | | |
| 6-89 | Me | Me | - (CH₂)₂O(CH₂)₂ - | | |
| 6-90 | Me | F | - (CH₂)₂O(CH₂)₂ - | | |
| 6-91 | Me | Cl | - (CH₂)₂O(CH₂)₂ - | | |
| 6-92 | Me | Br | - (CH₂)₂O(CH₂)₂ - | | |
| 6-93 | Me | I | - (CH₂)₂O(CH₂)₂ - | | |
| 6-94 | Me | CF₃ | - (CH₂)₂O(CH₂)₂ - | | |
| 6-95 | Me | CHF₂ | - (CH₂)₂O(CH₂)₂ - | | |
| 6-96 | Me | CF₂Cl | - (CH₂)₂O(CH₂)₂ - | | |
| 6-97 | Me | OMe | - (CH₂)₂O(CH₂)₂ - | | |
| 6-98 | Me | NO₂ | - (CH₂)₂O(CH₂)₂ - | | |
| 6-99 | Me | SO₂Me | - (CH₂)₂O(CH₂)₂ - | | |
| 6-100 | Cl | Me | - (CH₂)₂O(CH₂)₂ - | | |
| 6-101 | Cl | F | - (CH₂)₂O(CH₂)₂ - | | |
| 6-102 | Cl | Cl | - (CH₂)₂O(CH₂)₂ - | | (400 MHz, CDCl₃ δ, ppm) 7.44 (d,1H), 7.37 (d,1H), 4.35-4.31 (m,2H), 4.21 - 4.15 (m,2H), 3.55 (m,2H), 3.31 (m,2H), 2.49 (s,3H) |
| 6-103 | Cl | Br | - (CH₂)₂O(CH₂)₂ - | | |
| 6-104 | Cl | I | - (CH₂)₂O(CH₂)₂ - | | |
| 6-105 | Cl | CF₃ | - (CH₂)₂O(CH₂)₂ - | | |
| 6-106 | Cl | CHF₂ | - (CH₂)₂O(CH₂)₂ - | | |
| 6-107 | Cl | CF₂Cl | - (CH₂)₂O(CH₂)₂ - | | |
| 6-108 | Cl | OMe | - (CH₂)₂O(CH₂)₂ - | | |
| 6-109 | Cl | NO₂ | - (CH₂)₂O(CH₂)₂ - | | |
| 6-110 | Cl | SO₂Me | - (CH₂)₂O(CH₂)₂ - | | |
| 6-111 | OMe | Me | - (CH₂)₂O(CH₂)₂ - | | |
| 6-112 | OMe | F | - (CH₂)₂O(CH₂)₂ - | | |
| 6-113 | OMe | Cl | - (CH₂)₂O(CH₂)₂ - | | |
| 6-114 | OMe | Br | - (CH₂)₂O(CH₂)₂ - | | |
| 6-115 | OMe | I | - (CH₂)₂O(CH₂)₂ - | | |
| 6-116 | OMe | CF₃ | - (CH₂)₂O(CH₂)₂ - | | |
| 6-117 | OMe | CHF₂ | - (CH₂)₂O(CH₂)₂ - | | |
| 6-118 | OMe | CF₂Cl | - (CH₂)₂O(CH₂)₂ - | | |
| 6-119 | OMe | OMe | - (CH₂)₂O(CH₂)₂ - | | |
| 6-120 | OMe | NO₂ | - (CH₂)₂O(CH₂)₂ - | | |
| 6-121 | OMe | SO₂Me | - (CH₂)₂O(CH₂)₂ - | | |
| 6-122 | SO₂Me | Me | - (CH₂)₂O(CH₂)₂ - | | |
| 6-123 | SO₂Me | F | - (CH₂)₂O(CH₂)₂ - | | |
| 6-124 | SO₂Me | Cl | - (CH₂)₂O(CH₂)₂ - | | |
| 6-125 | SO₂Me | Br | - (CH₂)₂O(CH₂)₂ - | | |
| 6-126 | SO₂Me | I | - (CH₂)₂O(CH₂)₂ - | | |
| 6-127 | SO₂Me | CF₃ | - (CH₂)₂O(CH₂)₂ - | | |
| 6-128 | SO₂Me | CHF₂ | - (CH₂)₂O(CH₂)₂ - | | |
| 6-129 | SO₂Me | CF₂Cl | - (CH₂)₂O(CH₂)₂ - | | |
| 6-130 | SO₂Me | OMe | - (CH₂)₂O(CH₂)₂ - | | |
| 6-131 | SO₂Me | NO₂ | - (CH₂)₂O(CH₂)₂ - | | |
| 6-132 | SO₂Me | SO₂Me | - (CH₂)₂O(CH₂)₂ - | | |
| 6-133 | Cl | COOMe | Et | Et | |
| 6-134 | Cl | COOMe | - (CH₂)₅ - | | |
| 6-135 | Cl | COOMe | - (CH₂)₂O(CH₂)₂ - | | |

**Tabelle 7: Erfindungsgemäße Verbindungen der allgemeinen Formel (I), worin Q für Q4 und R^{z} für eine Methylgruppe stehen sowie R" und W jeweils Wasserstoff bedeuten**

| | | | | | |
|---|---|---|---|---|---|
| 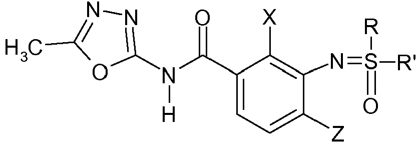 | | | | | |

| Nr. | X | Z | R | R' | Physikalische Daten (¹H-NMR) |
|---|---|---|---|---|---|
| 7-1 | Me | Me | Et | Et | |
| 7-2 | Me | F | Et | Et | |
| 7-3 | Me | Cl | Et | Et | |
| 7-4 | Me | Br | Et | Et | |
| 7-5 | Me | I | Et | Et | |
| 7-6 | Me | CF₃ | Et | Et | |
| 7-7 | Me | CHF₂ | Et | Et | |
| 7-8 | Me | CF₂Cl | Et | Et | |
| 7-9 | Me | OMe | Et | Et | |
| 7-10 | Me | NO₂ | Et | Et | |
| 7-11 | Me | SO₂Me | Et | Et | |
| 7-12 | Cl | Me | Et | Et | |
| 7-13 | Cl | F | Et | Et | |
| 7-14 | Cl | Cl | Et | Et | (400 MHz, CDCl₃ δ, ppm) 7.39 (d,1H), 7.31 (d,1H), 3.31 (q,4H), 2.55 (s,3H), 1.49 (t,6H) |
| 7-15 | Cl | Br | Et | Et | |
| 7-16 | Cl | I | Et | Et | |
| 7-17 | Cl | CF₃ | Et | Et | |
| 7-18 | Cl | CHF₂ | Et | Et | |
| 7-19 | Cl | CF₂Cl | Et | Et | |
| 7-20 | Cl | OMe | Et | Et | |
| 7-21 | Cl | NO₂ | Et | Et | |
| 7-22 | Cl | SO₂Me | Et | Et | |
| 7-23 | OMe | Me | Et | Et | |
| 7-24 | OMe | F | Et | Et | |
| 7-25 | OMe | Cl | Et | Et | |
| 7-26 | OMe | Br | Et | Et | |
| 7-27 | OMe | I | Et | Et | |
| 7-28 | OMe | CF₃ | Et | Et | |
| 7-29 | OMe | CHF₂ | Et | Et | |
| 7-30 | OMe | CF₂Cl | Et | Et | |
| 7-31 | OMe | OMe | Et | Et | |
| 7-32 | OMe | NO₂ | Et | Et | |
| 7-33 | OMe | SO₂Me | Et | Et | |
| 7-34 | SO₂Me | Me | Et | Et | |
| 7-35 | SO₂Me | F | Et | Et | |
| 7-36 | SO₂Me | Cl | Et | Et | |
| 7-37 | SO₂Me | Br | Et | Et | |
| 7-38 | SO₂Me | I | Et | Et | |
| 7-39 | SO₂Me | CF₃ | Et | Et | |
| 7-40 | SO₂Me | CHF₂ | Et | Et | |
| 7-41 | SO₂Me | CF₂Cl | Et | Et | |
| 7-42 | SO₂Me | OMe | Et | Et | |
| 7-43 | SO₂Me | NO₂ | Et | Et | |
| 7-44 | SO₂Me | SO₂Me | Et | Et | |
| 7-45 | Me | Me | - (CH₂)₅ - | | |
| 7-46 | Me | F | - (CH₂)₅ - | | |
| 7-47 | Me | Cl | - (CH₂)₅ - | | |
| 7-48 | Me | Br | - (CH₂)₅ - | | |
| 7-49 | Me | I | - (CH₂)₅ - | | |
| 7-50 | Me | CF₃ | - (CH₂)₅ - | | |
| 7-51 | Me | CHF₂ | - (CH₂)₅ - | | |
| 7-52 | Me | CF₂Cl | - (CH₂)₅ - | | |
| 7-53 | Me | OMe | - (CH₂)₅ - | | |
| 7-54 | Me | NO₂ | - (CH₂)₅ - | | |
| 7-55 | Me | SO₂Me | - (CH₂)₅ - | | |
| 7-56 | Cl | Me | - (CH₂)₅ - | | |
| 7-57 | Cl | F | - (CH₂)₅ - | | |
| 7-58 | Cl | Cl | - (CH₂)₅ - | | (400 MHz, CDCl₃ δ, ppm) 7.35 (d,1H), 3.49 (m,2H), 3.33 - 3.23 (m,2H), 2.57 (s,3H) |
| 7-59 | Cl | Br | - (CH₂)₅ - | | |
| 7-60 | Cl | I | - (CH₂)₅ - | | |
| 7-61 | Cl | CF₃ | - (CH₂)₅ - | | |
| 7-62 | Cl | CHF₂ | - (CH₂)₅ - | | |
| 7-63 | Cl | CF₂Cl | - (CH₂)₅ - | | |
| 7-64 | Cl | OMe | - (CH₂)₅ - | | |
| 7-65 | Cl | NO₂ | - (CH₂)₅ - | | |
| 7-66 | Cl | SO₂Me | - (CH₂)₅ - | | |
| 7-67 | OMe | Me | - (CH₂)₅ - | | |
| 7-68 | OMe | F | - (CH₂)₅ - | | |
| 7-69 | OMe | Cl | - (CH₂)₅ - | | |
| 7-70 | OMe | Br | - (CH₂)₅ - | | |
| 7-71 | OMe | I | - (CH₂)₅ - | | |
| 7-72 | OMe | CF₃ | - (CH₂)₅ - | | |
| 7-73 | OMe | CHF₂ | - (CH₂)₅ - | | |
| 7-74 | OMe | CF₂Cl | - (CH₂)₅ - | | |
| 7-75 | OMe | OMe | - (CH₂)₅ - | | |
| 7-76 | OMe | NO₂ | - (CH₂)₅ - | | |
| 7-77 | OMe | SO₂Me | - (CH₂)₅ - | | |
| 7-78 | SO₂Me | Me | - (CH₂)₅ - | | |
| 7-79 | SO₂Me | F | - (CH₂)₅ - | | |
| 7-80 | SO₂Me | Cl | - (CH₂)₅ - | | |
| 7-81 | SO₂Me | Br | - (CH₂)₅ - | | |
| 7-82 | SO₂Me | I | - (CH₂)₅ - | | |
| 7-83 | SO₂Me | CF₃ | - (CH₂)₅ - | | |
| 7-84 | SO₂Me | CHF₂ | - (CH₂)₅ - | | |
| 7-85 | SO₂Me | CF₂Cl | - (CH₂)₅ - | | |
| 7-86 | SO₂Me | OMe | - (CH₂)₅ - | | |
| 7-87 | SO₂Me | NO₂ | - (CH₂)₅ - | | |
| 7-88 | SO₂Me | SO₂Me | - (CH₂)₅ - | | |
| 7-89 | Me | Me | - (CH₂)₂O(CH₂)₂ - | | |
| 7-90 | Me | F | - (CH₂)₂O(CH₂)₂ - | | |
| 7-91 | Me | Cl | - (CH₂)₂O(CH₂)₂ - | | |
| 7-92 | Me | Br | - (CH₂)₂O(CH₂)₂ - | | |
| 7-93 | Me | I | - (CH₂)₂O(CH₂)₂ - | | |
| 7-94 | Me | CF₃ | - (CH₂)₂O(CH₂)₂ - | | |
| 7-95 | Me | CHF₂ | - (CH₂)₂O(CH₂)₂ - | | |
| 7-96 | Me | CF₂Cl | - (CH₂)₂O(CH₂)₂ - | | |
| 7-97 | Me | OMe | - (CH₂)₂O(CH₂)₂ - | | |
| 7-98 | Me | NO₂ | - (CH₂)₂O(CH₂)₂ - | | |
| 7-99 | Me | SO₂Me | - (CH₂)₂O(CH₂)₂ - | | |
| 7-100 | Cl | Me | - (CH₂)₂O(CH₂)₂ - | | |
| 7-101 | Cl | F | - (CH₂)₂O(CH₂)₂ - | | |
| 7-102 | Cl | Cl | - (CH₂)₂O(CH₂)₂ - | | (400 MHz, CDCl₃ δ, ppm) 7.41 (d,1H), 7.36 (d,1H), 4.32 (m,2H), 4.19 (m,2H), 3.57 (m,2H), 3.31 (m,2H), 2.55 (s,3H) |
| 7-103 | Cl | Br | - (CH₂)₂O(CH₂)₂ - | | |
| 7-104 | Cl | I | - (CH₂)₂O(CH₂)₂ - | | |
| 7-105 | Cl | CF₃ | - (CH₂)₂O(CH₂)₂ - | | |
| 7-106 | Cl | CHF₂ | - (CH₂)₂O(CH₂)₂ - | | |
| 7-107 | Cl | CF₂Cl | - (CH₂)₂O(CH₂)₂ - | | |
| 7-108 | Cl | OMe | - (CH₂)₂O(CH₂)₂ - | | |
| 7-109 | Cl | NO₂ | - (CH₂)₂O(CH₂)₂ - | | |
| 7-110 | Cl | SO₂Me | - (CH₂)₂O(CH₂)₂ - | | |
| 7-111 | OMe | Me | - (CH₂)₂O(CH₂)₂ - | | |
| 7-112 | OMe | F | - (CH₂)₂O(CH₂)₂ - | | |
| 7-113 | OMe | Cl | - (CH₂)₂O(CH₂)₂ - | | |
| 7-114 | OMe | Br | - (CH₂)₂O(CH₂)₂ - | | |
| 7-115 | OMe | I | - (CH₂)₂O(CH₂)₂ - | | |
| 7-116 | OMe | CF₃ | - (CH₂)₂O(CH₂)₂ - | | |
| 7-117 | OMe | CHF₂ | - (CH₂)₂O(CH₂)₂ - | | |
| 7-118 | OMe | CF₂Cl | - (CH₂)₂O(CH₂)₂ - | | |
| 7-119 | OMe | OMe | - (CH₂)₂O(CH₂)₂ - | | |
| 7-120 | OMe | NO₂ | - (CH₂)₂O(CH₂)₂ - | | |
| 7-121 | OMe | SO₂Me | - (CH₂)₂O(CH₂)₂ - | | |
| 7-122 | SO₂Me | Me | - (CH₂)₂O(CH₂)₂ - | | |
| 7-123 | SO₂Me | F | - (CH₂)₂O(CH₂)₂ - | | |
| 7-124 | SO₂Me | Cl | - (CH₂)₂O(CH₂)₂ - | | |
| 7-125 | SO₂Me | Br | - (CH₂)₂O(CH₂)₂ - | | |
| 7-126 | SO₂Me | I | - (CH₂)₂O(CH₂)₂ - | | |
| 7-127 | SO₂Me | CF₃ | - (CH₂)₂O(CH₂)₂ - | | |
| 7-128 | SO₂Me | CHF₂ | - (CH₂)₂O(CH₂)₂ - | | |
| 7-129 | SO₂Me | CF₂Cl | - (CH₂)₂O(CH₂)₂ - | | |
| 7-130 | SO₂Me | OMe | - (CH₂)₂O(CH₂)₂ - | | |
| 7-131 | SO₂Me | NO₂ | - (CH₂)₂O(CH₂)₂ - | | |
| 7-132 | SO₂Me | SO₂Me | - (CH₂)₂O(CH₂)₂ - | | |
| 7-133 | Cl | COOMe | Et | Et | |
| 7-134 | Cl | COOMe | - (CH₂)₅ - | | |
| 7-135 | Cl | COOMe | - (CH₂)₂O(CH₂)₂ - | | |

**Tabelle 8: Erfindungsgemäße Verbindungen der allgemeinen Formel (I) in Form der Natriumsalze, worin Q für Q1 und R^{x} für eine Methylgruppe stehen sowie W Wasserstoff bedeutet**

| | | | | | |
|---|---|---|---|---|---|
| 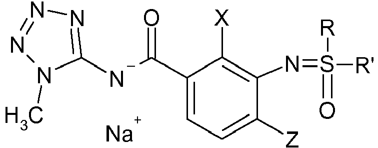 | | | | | |

| Nr. | X | Z | R | R' | Physikalische Daten (¹H-NMR) |
|---|---|---|---|---|---|
| 8-1 | Me | Me | Et | Et | |
| 8-2 | Me | Cl | Et | Et | |
| 8-3 | Me | CF₃ | Et | Et | |
| 8-4 | Me | CHF₂ | Et | Et | |
| 8-5 | Cl | Me | Et | Et | |
| 8-6 | Cl | Cl | Et | Et | |
| 8-7 | Cl | CF₃ | Et | Et | |
| 8-8 | Cl | CHF₂ | Et | Et | |
| 8-9 | OMe | Me | Et | Et | |
| 8-10 | OMe | Cl | Et | Et | |
| 8-11 | OMe | CF₃ | Et | Et | |
| 8-12 | OMe | CHF₂ | Et | Et | |
| 8-13 | SO₂Me | Me | Et | Et | |
| 8-14 | SO₂Me | Cl | Et | Et | |
| 8-15 | SO₂Me | CF₃ | Et | Et | |
| 8-16 | SO₂Me | CHF₂ | Et | Et | |
| 8-17 | Me | Me | - (CH₂)₅ - | | |
| 8-18 | Me | Cl | - (CH₂)₅ - | | |
| 8-19 | Me | CF₃ | - (CH₂)₅ - | | |
| 8-20 | Me | CHF₂ | - (CH₂)₅ - | | |
| 8-21 | Cl | Me | - (CH₂)₅ - | | |
| 8-22 | Cl | Cl | - (CH₂)₅ - | | |
| 8-23 | Cl | CF₃ | - (CH₂)₅ - | | |
| 8-24 | Cl | CHF₂ | - (CH₂)₅ - | | |
| 8-25 | OMe | Me | - (CH₂)₅ - | | |
| 8-26 | OMe | Cl | - (CH₂)₅ - | | |
| 8-27 | OMe | CF₃ | - (CH₂)₅ - | | |
| 8-28 | OMe | CHF₂ | - (CH₂)₅ - | | |
| 8-29 | SO₂Me | Me | - (CH₂)₅ - | | |
| 8-30 | SO₂Me | Cl | - (CH₂)₅ - | | |
| 8-31 | SO₂Me | CF₃ | - (CH₂)₅ - | | |
| 8-32 | SO₂Me | CHF₂ | - (CH₂)₅ - | | |
| 8-33 | Me | Me | - (CH₂)₂O(CH₂)₂ - | | |
| 8-34 | Me | Cl | - (CH₂)₂O(CH₂)₂ - | | |
| 8-35 | Me | CF₃ | - (CH₂)₂O(CH₂)₂ - | | |
| 8-36 | Me | CHF₂ | - (CH₂)₂O(CH₂)₂ - | | |
| 8-37 | Cl | Me | - (CH₂)₂O(CH₂)₂ - | | |
| 8-38 | Cl | Cl | - (CH₂)₂O(CH₂)₂ - | | |
| 8-39 | Cl | CF₃ | - (CH₂)₂O(CH₂)₂ - | | |
| 8-40 | Cl | CHF₂ | - (CH₂)₂O(CH₂)₂ - | | |
| 8-41 | OMe | Me | - (CH₂)₂O(CH₂)₂ - | | |
| 8-42 | OMe | Cl | - (CH₂)₂O(CH₂)₂ - | | |
| 8-43 | OMe | CF₃ | - (CH₂)₂O(CH₂)₂ - | | |
| 8-44 | OMe | CHF₂ | - (CH₂)₂O(CH₂)₂ - | | |
| 8-45 | SO₂Me | Me | - (CH₂)₂O(CH₂)₂ - | | |
| 8-46 | SO₂Me | Cl | - (CH₂)₂O(CH₂)₂ - | | |
| 8-47 | SO₂Me | CF₃ | - (CH₂)₂O(CH₂)₂ - | | |
| 8-48 | SO₂Me | CHF₂ | - (CH₂)₂O(CH₂)₂ - | | |
| 8-49 | Cl | COOMe | Et | Et | |
| 8-50 | Cl | COOMe | - (CH₂)₅ - | | |
| 8-51 | Cl | COOMe | - (CH₂)₂O(CH₂)₂ - | | |

**Tabelle 9: Erfindungsgemäße Verbindungen der allgemeinen Formel (I), worin Q für Q3 und R^{y} für Chlor stehen sowie R" und W jeweils Wasserstoff bedeuten**

| | | | | | |
|---|---|---|---|---|---|
| 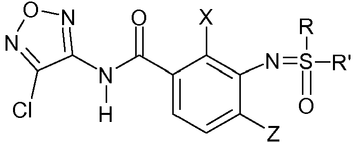 | | | | | |

| Nr. | X | Z | R | R' | Physikalische Daten (¹H-NMR) |
|---|---|---|---|---|---|
| 9-1 | Me | Me | Et | Et | |
| 9-2 | Me | F | Et | Et | |
| 9-3 | Me | Cl | Et | Et | |
| 9-4 | Me | Br | Et | Et | |
| 9-5 | Me | I | Et | Et | |
| 9-6 | Me | CF₃ | Et | Et | |
| 9-7 | Me | CHF₂ | Et | Et | |
| 9-8 | Me | CF₂Cl | Et | Et | |
| 9-9 | Me | OMe | Et | Et | |
| 9-10 | Me | NO₂ | Et | Et | |
| 9-11 | Me | SO₂Me | Et | Et | |
| 9-12 | Cl | Me | Et | Et | |
| 9-13 | Cl | F | Et | Et | |
| 9-14 | Cl | Cl | Et | Et | (400 MHz, CDCl₃ δ, ppm) 7.49 (d,1H), 7.46 (d,1H), 3.29 (q,4H), 1.51 (t,6H) |
| 9-15 | Cl | Br | Et | Et | |
| 9-16 | Cl | I | Et | Et | |
| 9-17 | Cl | CF₃ | Et | Et | |
| 9-18 | Cl | CHF₂ | Et | Et | |
| 9-19 | Cl | CF₂Cl | Et | Et | |
| 9-20 | Cl | OMe | Et | Et | |
| 9-21 | Cl | NO₂ | Et | Et | |
| 9-22 | Cl | SO₂Me | Et | Et | |
| 9-23 | OMe | Me | Et | Et | |
| 9-24 | OMe | F | Et | Et | |
| 9-25 | OMe | Cl | Et | Et | |
| 9-26 | OMe | Br | Et | Et | |
| 9-27 | OMe | I | Et | Et | |
| 9-28 | OMe | CF₃ | Et | Et | |
| 9-29 | OMe | CHF₂ | Et | Et | |
| 9-30 | OMe | CF₂Cl | Et | Et | |
| 9-31 | OMe | OMe | Et | Et | |
| 9-32 | OMe | NO₂ | Et | Et | |
| 9-33 | OMe | SO₂Me | Et | Et | |
| 9-34 | SO₂Me | Me | Et | Et | |
| 9-35 | SO₂Me | F | Et | Et | |
| 9-36 | SO₂Me | Cl | Et | Et | |
| 9-37 | SO₂Me | Br | Et | Et | |
| 9-38 | SO₂Me | I | Et | Et | |
| 9-39 | SO₂Me | CF₃ | Et | Et | |
| 9-40 | SO₂Me | CHF₂ | Et | Et | |
| 9-41 | SO₂Me | CF₂Cl | Et | Et | |
| 9-42 | SO₂Me | OMe | Et | Et | |
| 9-43 | SO₂Me | NO₂ | Et | Et | |
| 9-44 | SO₂Me | SO₂Me | Et | Et | |
| 9-45 | Me | Me | - (CH₂)₅ - | | |
| 9-46 | Me | F | - (CH₂)₅ - | | |
| 9-47 | Me | Cl | - (CH₂)₅ - | | |
| 9-48 | Me | Br | - (CH₂)₅ - | | |
| 9-49 | Me | I | - (CH₂)₅ - | | |
| 9-50 | Me | CF₃ | - (CH₂)₅ - | | |
| 9-51 | Me | CHF₂ | - (CH₂)₅ - | | |
| 9-52 | Me | CF₂Cl | - (CH₂)₅ - | | |
| 9-53 | Me | OMe | - (CH₂)₅ - | | |
| 9-54 | Me | NO₂ | - (CH₂)₅ - | | |
| 9-55 | Me | SO₂Me | - (CH₂)₅ - | | |
| 9-56 | Cl | Me | - (CH₂)₅ - | | |
| 9-57 | Cl | F | - (CH₂)₅ - | | |
| 9-58 | Cl | Cl | -(CH₂)₅- | | (400 MHz, CDCl₃ δ, ppm) 7.48 (d,1H), 7.45 (d, 1H), 3.40 - 3.25 (m,4H), 2.30 - 2.11 (m,4H), 1.88 - 1.80 (m, 1H), 1.70-1.53 (m,1H) |
| 9-59 | Cl | Br | -(CH₂)₅- | | |
| 9-60 | Cl | I | -(CH₂)₅- | | |
| 9-61 | Cl | CF₃ | -(CH₂)₅- | | |
| 9-62 | Cl | CHF₂ | -(CH₂)₅- | | |
| 9-63 | Cl | CF₂Cl | -(CH₂)₅- | | |
| 9-64 | Cl | OMe | -(CH₂)₅- | | |
| 9-65 | Cl | NO₂ | -(CH₂)₅- | | |
| 9-66 | Cl | SO₂Me | -(CH₂)₅- | | |
| 9-67 | OMe | Me | -(CH₂)₅- | | |
| 9-68 | OMe | F | -(CH₂)₅- | | |
| 9-69 | OMe | Cl | -(CH₂)₅- | | |
| 9-70 | OMe | Br | -(CH₂)₅- | | |
| 9-71 | OMe | I | -(CH₂)₅- | | |
| 9-72 | OMe | CF₃ | -(CH₂)₅- | | |
| 9-73 | OMe | CHF₂ | -(CH₂)₅- | | |
| 9-74 | OMe | CF₂Cl | -(CH₂)₅- | | |
| 9-75 | OMe | OMe | -(CH₂)₅- | | |
| 9-76 | OMe | NO₂ | -(CH₂)₅- | | |
| 9-77 | OMe | SO₂Me | -(CH₂)₅- | | |
| 9-78 | SO₂Me | Me | -(CH₂)₅- | | |
| 9-79 | SO₂Me | F | -(CH₂)₅- | | |
| 9-80 | SO₂Me | Cl | -(CH₂)₅- | | |
| 9-81 | SO₂Me | Br | -(CH₂)₅- | | |
| 9-82 | SO₂Me | I | -(CH₂)₅- | | |
| 9-83 | SO₂Me | CF₃ | -(CH₂)₅- | | |
| 9-84 | SO₂Me | CHF₂ | -(CH₂)₅- | | |
| 9-85 | SO₂Me | CF₂Cl | -(CH₂)₅- | | |
| 9-86 | SO₂Me | OMe | -(CH₂)₅- | | |
| 9-87 | SO₂Me | NO₂ | -(CH₂)₅- | | |
| 9-88 | SO₂Me | SO₂Me | -(CH₂)₅- | | |
| 9-89 | Me | Me | -(CH₂)₂O(CH₂)₂- | | |
| 9-90 | Me | F | -(CH₂)₂O(CH₂)₂- | | |
| 9-91 | Me | Cl | -(CH₂)₂O(CH₂)₂- | | |
| 9-92 | Me | Br | -(CH₂)₂O(CH₂)₂- | | |
| 9-93 | Me | I | -(CH₂)₂O(CH₂)₂- | | |
| 9-94 | Me | CF₃ | -(CH₂)₂O(CH₂)₂- | | |
| 9-95 | Me | CHF₂ | -(CH₂)₂O(CH₂)₂- | | |
| 9-96 | Me | CF₂Cl | -(CH₂)₂O(CH₂)₂- | | |
| 9-97 | Me | OMe | -(CH₂)₂O(CH₂)₂- | | |
| 9-98 | Me | NO₂ | -(CH₂)₂O(CH₂)₂- | | |
| 9-99 | Me | SO₂Me | -(CH₂)₂O(CH₂)₂- | | |
| 9-100 | Cl | Me | -(CH₂)₂O(CH₂)₂- | | |
| 9-101 | Cl | F | -(CH₂)₂O(CH₂)₂- | | |
| 9-102 | Cl | Cl | - (CH₂)₂O(CH₂)₂- | | (400 MHz, DMSO-d₆ δ, ppm) 7.57 (d,1H), 7.27 (d,1H), 4.23 - 4.12 (m,2H), 4.06 - 3.97 (m,2H), 3.55 - 3.40 (m,4H) |
| 9-103 | Cl | Br | -(CH₂)₂O(CH₂)₂- | | |
| 9-104 | Cl | I | -(CH₂)₂O(CH₂)₂- | | |
| 9-105 | Cl | CF₃ | -(CH₂)₂O(CH₂)₂- | | |
| 9-106 | Cl | CHF₂ | -(CH₂)₂O(CH₂)₂- | | |
| 9-107 | Cl | CF₂Cl | -(CH₂)₂O(CH₂)₂- | | |
| 9-108 | Cl | OMe | -(CH₂)₂O(CH₂)₂- | | |
| 9-109 | Cl | NO₂ | -(CH₂)₂O(CH₂)₂- | | |
| 9-110 | Cl | SO₂Me | -(CH₂)₂O(CH₂)₂- | | |
| 9-111 | OMe | Me | -(CH₂)₂O(CH₂)₂- | | |
| 9-112 | OMe | F | -(CH₂)₂O(CH₂)₂- | | |
| 9-113 | OMe | Cl | -(CH₂)₂O(CH₂)₂- | | |
| 9-114 | OMe | Br | -(CH₂)₂O(CH₂)₂- | | |
| 9-115 | OMe | I | -(CH₂)₂O(CH₂)₂- | | |
| 9-116 | OMe | CF₃ | -(CH₂)₂O(CH₂)₂- | | |
| 9-117 | OMe | CHF₂ | -(CH₂)₂O(CH₂)₂- | | |
| 9-118 | OMe | CF₂Cl | -(CH₂)₂O(CH₂)₂- | | |
| 9-119 | OMe | OMe | -(CH₂)₂O(CH₂)₂- | | |
| 9-120 | OMe | NO₂ | -(CH₂)₂O(CH₂)₂- | | |
| 9-121 | OMe | SO₂Me | -(CH₂)₂O(CH₂)₂- | | |
| 9-122 | SO₂Me | Me | -(CH₂)₂O(CH₂)₂- | | |
| 9-123 | SO₂Me | F | -(CH₂)₂O(CH₂)₂- | | |
| 9-124 | SO₂Me | Cl | -(CH₂)₂O(CH₂)₂- | | |
| 9-125 | SO₂Me | Br | -(CH₂)₂O(CH₂)₂- | | |
| 9-126 | SO₂Me | I | -(CH₂)₂O(CH₂)₂- | | |
| 9-127 | SO₂Me | CF₃ | -(CH₂)₂O(CH₂)₂- | | |
| 9-128 | SO₂Me | CHF₂ | -(CH₂)₂O(CH₂)₂- | | |
| 9-129 | SO₂Me | CF₂Cl | -(CH₂)₂O(CH₂)₂- | | |
| 9-130 | SO₂Me | OMe | -(CH₂)₂O(CH₂)₂- | | |
| 9-131 | SO₂Me | NO₂ | -(CH₂)₂O(CH₂)₂- | | |
| 9-132 | SO₂Me | SO₂Me | -(CH₂)₂O(CH₂)₂- | | |
| 9-133 | Cl | COOMe | Et | Et | |
| 9-134 | Cl | COOMe | -(CH₂)₅- | | |
| 9-135 | Cl | COOMe | -(CH₂)₂O(CH₂)₂- | | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 6-102, 6-014 und 6-058 bei einer Aufwandmenge von 320 g/ha jeweils eine mindestens 80%-ige Wirkung gegen Abutilon theophrasti, Amaranthus retroflexus und Matricaria inodora.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen zahlreiche erfindungsgemäße Verbindungen bei einer Aufwandmenge von 80 g/ha jeweils eine hervorragende Wirkung gegen eine Vielzahl unerwünschter Pflanzen.

### 3. Vergleichsversuche

Entsprechend den genannten Bedingungen zur Prüfung der herbiziden Wirkung gegen Schadpflanzen im Vorauflauf wurden Vergleichsversuche zwischen erfindungsgemäßen und aus WO 2011/035874 A1 bekannten Verbindungen durchgeführt. Die Versuchergebnisse zeigen, dass die erfindungsgemäßen Verbindungen eine überlegene Wirkung gegen zahlreiche Schadpflanzen aufweisen.

Die hier verwendeten Abkürzungen bedeuten

| | | | |
|---|---|---|---|
| ABUTH | Abutilon theophrasti | AMARE | Amaranthus retroflexus |
| AVEFA | Avena fatua | MATIN | Matricaria inodora |
| PHBPU | Pharbitis purpureum | STEME | Stellaria media |
| VERPE | Veronica persica | | |

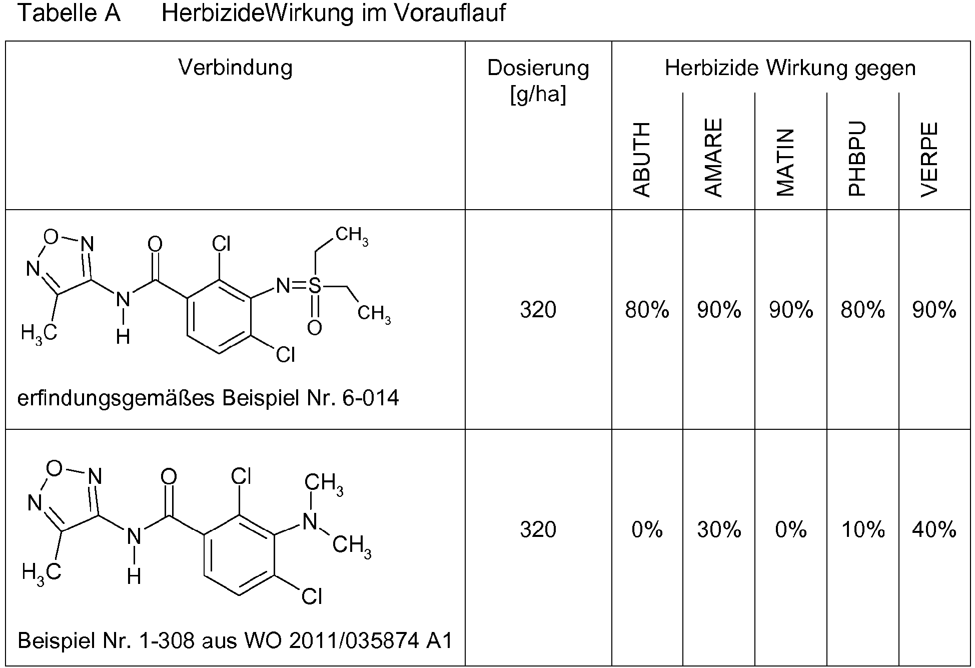

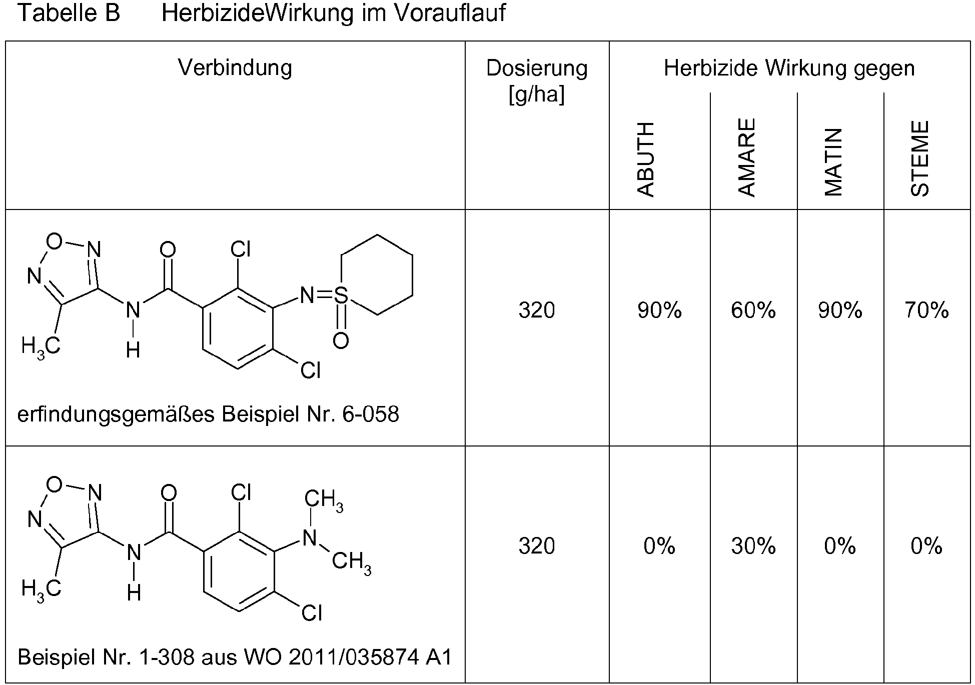

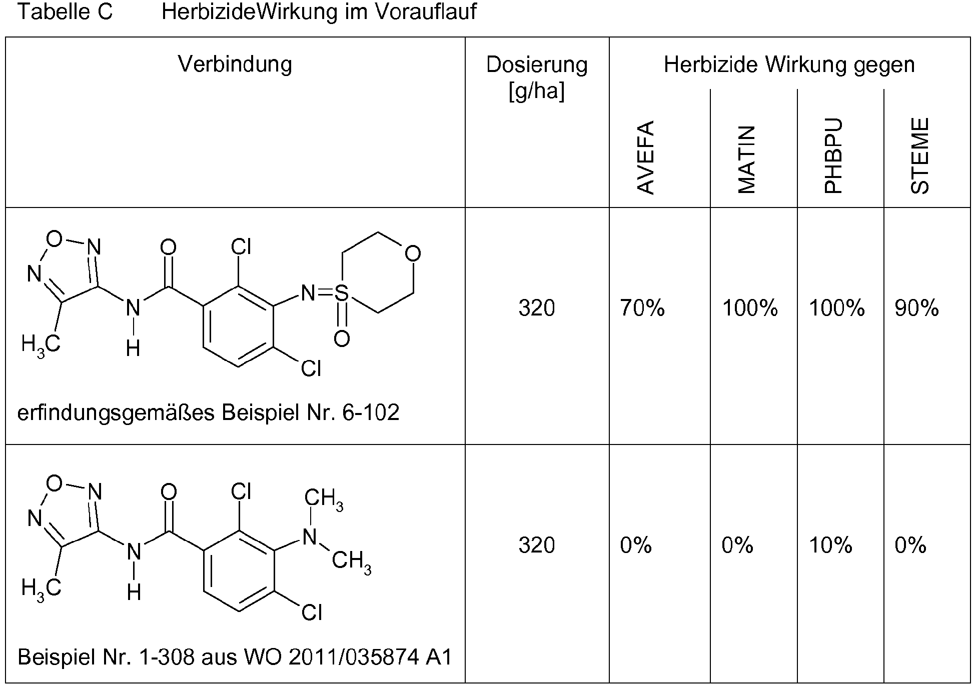

## Patentansprüche

1. Sulfinylaminobenzamide der Formel (I) oder deren Salze 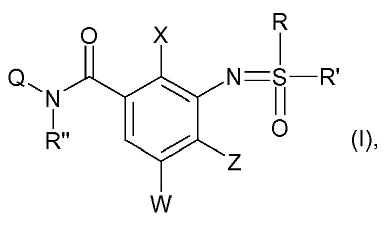 worin die Symbole und Indizes folgende Bedeutungen haben:
Q bedeutet einen Rest Q1, Q2, Q3 oder Q4, 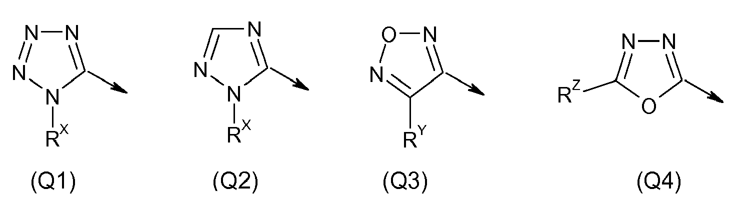
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)2N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Z bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO,R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
W bedeutet Wasserstoff, Halogen, Nitro, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, Halogen-(C₃-C₇)-Cycloalkyl, (C₁-C₆)-Alkoxy, Halogen-(C₁-C₆)-alkoxy, (C₁-C₆)-Alkyl-(O)ₙS-, (C₁-C₆)-Halogenalkyl-(O)ₙS-, (C₁-C₆)-Alkoxy-(C₁-C₄)-alkyl, (C₁-C₆)-Alkoxy-(C₁-C₄)-halogenalkyl, R¹(O)C, R¹(R¹ON=)C, R¹O(O)C, (R¹)₂N, R¹(O)C(R¹)N oder R²(O)₂S(R¹)N,
R und R' bedeuten unabhängig voneinander jeweils (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C,R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
oder R und R' bilden gemeinsam mit dem Schwefel-Atom, an dem sie gebunden sind,
einen 3- bis 8-gliedrigen ungesättigten, teilgesättigten oder gesättigten Ring, der außer den Kohlenstoffatomen und außer dem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R" bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C, R¹O(O)C, (R¹)₂N(O)C, R¹O, (R¹)₂N, R²O(O)C(R¹)N, (R¹)₂N(O)C(R¹)N, R²(O)₂S, oder jeweils durch s Reste aus der Gruppe bestehend aus Methyl, Ethyl, Methoxy, Nitro, Trifluormethyl und Halogen substituiertes Benzyl,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus Nitro, Cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)2S(R¹)N, (C₃-C₆)-Cycloalkyl, Heteroaryl, Heterocyclyl und Phenyl substituiert sind, und wobei die vier letztgenannten Reste durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl,
(C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfanyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl, und wobei Heterocyclyl n Oxogruppen trägt,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, R¹O, R¹(H)N, Methoxycarbonyl, Ethoxycarbonyl, Methylcarbonyl, Dimethylamino, Trifluormethylcarbonyl, Acetylamino, Methylsulfanyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl,R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl,
R⁷ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
n bedeutet 0, 1 oder 2,
m bedeutet 0, 1, 2, 3 oder 4,
s bedeutet 0, 1, 2 oder 3.

2. Sulfinylaminobenzamide nach Anspruch 1, worin
Q bedeutet einen Rest Q1, Q2, Q3 oder Q4, 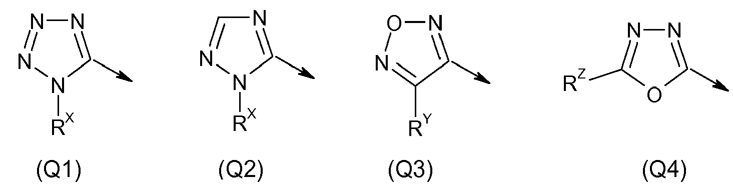
X bedeutet Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)2(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Z bedeutet Wasserstoff, Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
W bedeutet Wasserstoff, Halogen, Nitro, Cyano, (C₁-C₆)-Alkyl, Halogen-(C₁-C₆)-alkyl, (C₃-C₇)-Cycloalkyl, (C₁-C₆)-Alkoxy, (C₁-C₆)-Alkyl-(O)ₙS-, R¹O(O)C, (R¹)₂N, R¹(O)C(R¹)N oder R²(O)₂S(R¹)N,
R und R' bedeuten unabhängig voneinander jeweils (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-Cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, (C₁-C₆)-Alkyl, Halogen-(C₁-C₆)-alkyl, R¹O(O)C, (R¹)₂N(O)C, R¹O, (R¹)₂N, R²(O)ₙS, und R¹O-(C₁-C₆)-Alkyl substituiert sind, und wobei Heterocyclyl n Oxogruppen trägt,
oder R und R' bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außerdem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Halogen, (C₁-C₆)-Alkyl, Halogen-(C₁-C₆)-alkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R" bedeutet Wasserstoff,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy und Halogen substituiert sind, und wobei Heterocyclyl n Oxogruppen trägt,
oder R^{X} bedeutet (C₃-C₇)-Cycloalkyl, wobei dieser Rest jeweils durch s Reste aus der Gruppe bestehend aus Halogen, (C₁-C₆)-Alkyl und Halogen-(C₁-C₆)-alkyl substituiert ist,
R^{Y} bedeutet Wasserstoff, (C₁-C₆)-Alkyl, Halogen-(C₁-C₆)-alkyl, (C₃-C₇)-Cycloalkyl, (C₁-C₆)-Alkoxy, Methoxycarbonyl, Methoxycarbonylmethyl, Halogen, Amino, Aminocarbonyl oder Methoxymethyl,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, R¹O, R¹(H)N, Methoxycarbonyl, Acetylamino oder Methylsulfonyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R⁵ bedeutet Wasserstoff oder (C₁-C₄)-Alkyl,
R⁷ bedeutet Acetoxy, Acetamido, Methoxycarbonyl oder (C₃-C₆)-Cycloalkyl,
n bedeutet 0, 1 oder 2,
m bedeutet 0, 1 oder 2,
s bedeutet 0, 1, 2 oder 3.

3. Sulfinylaminobenzamide nach Anspruch 1 oder 2, worin
Q bedeutet einen Rest Q1, Q2, Q3 oder Q4, 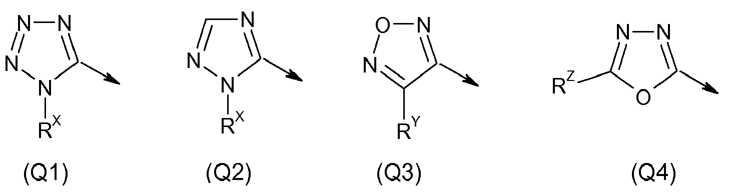
X bedeutet Nitro, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Hydroxycarbonyl, Methoxycarbonyl, Ethoxycarbonyl, Methoxy, Ethoxy, Methylsulfanyl, Methylsulfinyl, Methylsulfonyl, Methoxymethyl, Ethoxymethyl, Methoxyethyl, Methoxyethoxymethyl, Methylthiomethyl, Methylsulfinylmethyl oder Methylsulfonylmethyl,
Z bedeutet Wasserstoff, Nitro, Cyano, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Hydroxycarbonyl, Methoxycarbonyl, Ethoxycarbonyl, Methoxy, Ethoxy, Methylsulfanyl, Methylsulfinyl oder Methylsulfonyl,
W bedeutet Wasserstoff, Chlor oder Methyl,
R und R' bedeuten unabhängig voneinander jeweils Methyl, Ethyl oder n-Propyl, oder
R und R' bilden gemeinsam mit dem Schwefel-Atom, an dem sie gebunden sind, einen 5- oder 6-gliedrigen gesättigten Ring, der außer den Kohlenstoffatomen und außer dem Schwefelatom der Sulfoximinogruppe m Sauerstoffatome enthält,
R" bedeutet Wasserstoff,
R^{X} bedeutet Methyl, Ethyl, n-Propyl, Prop-2-en-1-yl, Methoxyethyl, Ethoxyethyl oder Methoxyethoxyethyl,
R^{Y} bedeutet Methyl, Ethyl, n-Propyl, Chlor oder Amino,
R^{Z} bedeutet Methyl, Ethyl, n-Propyl oder Methoxymethyl,
m bedeutet 0 oder 1.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend cyprosulfamid, cloquintocet-mexyl, mefenpyr-diethyl oder isoxadifen-ethyl.

9. Herbizide Mittel nach einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. Sulfinylaminobenzamides of the formula (I) or a salt thereof 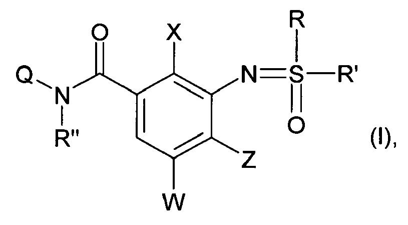 in which the symbols and indices are each defined as follows:
Q is a Q1, Q2, Q3 or Q4 radical, 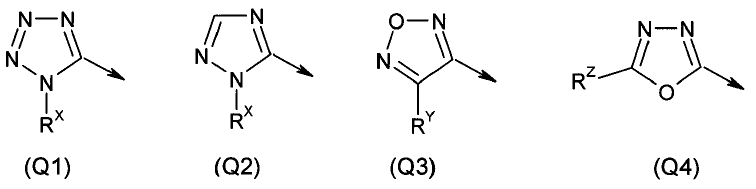
X is nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆) -alkynyl, halo- (C₃-C₆) -alkynyl, (C₃-C₆)-cycloalkyl, halo- (C₃-C₆) -cycloalkyl, (C₃-C₆) -cycloalkyl-(C₁-C₆)-alkyl, halo-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N (0) C, R¹(O)C(R¹)N(O)C, R²O(O)C(R¹)N(O)C, (R¹)₂N(O)C(R¹)N(O)C, R²(O)₂S(R¹) N (0) C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-alkyl, R¹O(O)C-(C₁-C₆)-alkyl, (R¹)₂N(O) C- (C₁-C₆) -alkyl, (R¹O)(R¹)N(O)C-(C₁-C₆) -alkyl, (R¹)₂N(R¹)N(O)C-(C₁-C₆)-alkyl, R¹(O)C(R¹)N(O)C-(C₁-C₆)-alkyl, R²O(O)C(R¹)N(O)C-(C₁-C₆)-alkyl, (R¹)₂N(O)C(R¹)N(O)C-(C₁-C₆)-alkyl, R²(O)₂S(R¹)N(O)C-(C₁-C₆)-alkyl, R¹O(O)₂S(R¹)N(O)C-(C₁-C₆)-alkyl, (R¹)₂N(O)₂S(R¹)N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R²O(O)CO-(C₁-C₆)-alkyl, (R¹)₂N(O) CO-(C₁-C₆)-alkyl, (R¹)₂N-(C₁-C₆)-alkyl, R¹(O)C(R¹)N- (C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R¹O(O)₂S(R¹)N- (C₁-C₆) -alkyl, (R¹)₂N(O)₂S(R¹)N-(C₁-C₆) -alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R²O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O**-**(C₁-C₆)-alkyl, and where heterocyclyl bears n oxo groups,
Z is hydrogen, nitro, halogen, cyano, thiocyanato, (C₁-C₆)-alkyl, halo- (C₁-C₆) -alkyl, (C₂-C₆)-alkenyl, halo-(C₂-C₆)-alkenyl, (C₂-C₆)-alkynyl, halo- (C₃-C₆) -alkynyl, (C₃-C₆)-cycloalkyl, halo- (C₃-C₆) -cycloalkyl, (C₃-C₆)-cycloalkyl- (C₁-C₆) -alkyl, halo- (C₃-C₆) -cycloalkyl- (C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, (R¹O)(R¹)N(O)C-(C₁-C₆)-alkyl, (R¹)₂N(R¹)N(O)C-(C₁-C₆)-alkyl, R¹(O)C(R¹)N(O)C-(C₁-C₆)-alkyl, R²O(O)C(R¹)N(O)C-(C₁-C₆)-alkyl, (R¹)₂N(O)C(R¹)N(O)C-(C₁-C₆)-alkyl, R²(O)₂S(R¹)N(O)C-(C₁-C₆)-alkyl, R¹O(O)₂S(R¹)N(O)C-(C₁-C₆)-alkyl, (R¹)₂N(O)₂S(R¹)N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R²O(O)CO-(C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R¹O(O)₂S(R¹)N-(C₁-C₆)-alkyl, (R¹)₂N(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R²O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
W is hydrogen, halogen, nitro, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₇)-cycloalkyl, halo-(C₃-C₇)-cycloalkyl, (C₁-C₆)-alkoxy, halo-(C₁-C₆)-alkoxy, (C₁-C₆)-alkyl-(O)ₙS-, (C₁-C₆)-haloalkyl-(O)ₙS-, (C₁-C₆)-alkoxy-(C₁-C₄)-alkyl, (C₁-C₆)-alkoxy-(C₁-C₄)-haloalkyl, R¹(O)C, R¹(R¹ON=)C, R¹O(O)C, (R¹)₂N, R¹(O)C(R¹)N or R²(O)₂S(R¹)N,
R and R' are each independently (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, (C₁-C₆)-alkoxy-(C₁-C₆)-alkyl, halo-(C₁-C₆)-alkoxy-(C₁-C₆)-alkyl, phenyl, heteroaryl or heterocyclyl, where the three latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl bears n oxo groups, or R and R' together with the sulfur atom to which they are bonded form a 3- to 8-membered unsaturated, semisaturated or saturated ring which contains, apart from the carbon atoms and apart from the sulfur atom of the sulfoximino group, in each case m ring members from the group consisting of N(R¹), 0 and S(O)ₙ, and where this ring in each case is substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R₂(O)ₙS, R¹O(O)₂S, (R¹)₂N(O)₂S and R¹O-(C₁-C₆)-alkyl, and where this ring bears n oxo groups,
R'' is hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆-alkynyl, (C₃-C₆-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C-(C₁-C₆)-alkyl, R¹O(O)C-(C₁-C₆)-alkyl, (R¹)₂N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C, R¹O(O)C, (R¹)₂N(O)C, R¹O, (R¹)₂N, R²O(O)C(R¹)N, (R¹)₂N(O)C(R¹)N, R²(O)₂S, or benzyl substituted in each case by s radicals from the group consisting of methyl, ethyl, methoxy, nitro, trifluoromethyl and halogen,
R^{x} is (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, where the six aforementioned radicals are each substituted by s radicals from the group consisting of nitro, cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyl, heteroaryl, heterocyclyl and phenyl, and where the four latter radicals are substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl bears n oxo groups, or R^{x} is (C₃-C₇)-cycloalkyl, heteroaryl, heterocyclyl or phenyl, where the four aforementioned radicals are each substituted by s radicals from the group consisting of halogen, nitro, cyano, (C₁-C₆)-alkyl, halo-(C₁-C₆)-alkyl, (C₃-C₆)-cycloalkyl, (C₁-C₆)-alkyl-S(O)ₙ, (C₁-C₆)-alkoxy, halo-(C₁-C₆)-alkoxy and (C₁-C₆)-alkoxy-(C₁-C₄)-alkyl,
R^{Y} is hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, (C₁-C₆)-alkoxy, halo- (C₁-C₆) -alkoxy, (C₂-C₆)-alkenyloxy, (C₂-C₆)-alkynyloxy, cyano, nitro, methylsulfanyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or heteroaryl, heterocyclyl or phenyl each substituted by s radicals from the group consisting of (C₁-C₆)-alkyl, halo-(C₁-C₆)-alkyl, (C₁-C₆)-alkoxy, halo-(C₁-C₆₎-alkoxy and halogen, and where heterocyclyl bears n oxo groups,
R^{Z} is hydrogen, (C₁-C₆)-alkyl, R¹O-(C₁-C₆)-alkyl, R⁷CH₂, (C₃-C₇)-cycloalkyl, halo-(C₁-C₆)-alkyl, (C₂-C₆)-alkenyl, halo-(C₂-C₆)-alkenyl, (C₂-C₆)-alkynyl, halo- (C₃-C₆)-alkynyl, R¹O, R¹(H)N, methoxycarbonyl, ethoxycarbonyl, methylcarbonyl, dimethylamino, trifluoromethylcarbonyl, acetylamino, methylsulfanyl, methylsulfinyl, methylsulfonyl, or heteroaryl, heterocyclyl, benzyl oder phenyl each substituted by s radicals from the group consisting of halogen, nitro, cyano, (C₁-C₆)-alkyl, halo-(C₁-C₆)-alkyl, (C₃-C₆)-cycloalkyl, (C₁-C₆)-alkyl-S(O)ₙ, (C₁-C₆)-alkoxy, halo-(C₁-C₆)-alkoxy and (C₁-C₆)-alkoxy-(C₁-C₄)-alkyl, where heterocyclyl bears n oxo groups,
R¹ is hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, (C₃-C₆)-cycloalkenyl, halo-(C₃-C₆)-cycloalkyl, (C₃-C₆)-cycloalkyl-(C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, phenyl-N(R³)-(C₁-C₆)-alkyl, heteroaryl-N(R³)-(C₁-C₆)-alkyl, heterocyclyl-N(R³)-(C₁-C₆)-alkyl, phenyl-S(O)ₙ-(C₁-C₆)-alkyl, heteroaryl-S(O)ₙ-(C₁-C₆)-alkyl, heterocyclyl-S(O)ₙ-(C₁-C₆)-alkyl, where the fifteen latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O-(C₁-C₆)-alkyl, and where heterocyclyl bears n oxo groups,
R² is (C₁-C₆)-alkyl, halo-(C₁-C₆)-alkyl, (C₂-C₆)-alkenyl, halo- (C₂-C₆)-alkenyl, (C₂-C₆)-alkynyl, halo-(C₃-C₆)-alkynyl, (C₃-C₆)-cycloalkyl, (C₃-C₆)-cycloalkenyl, halo-(C₃-C₆)-cycloalkyl, (C₃-C₆)-cycloalkyl- (C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, phenyl-N(R³)-(C₁-C₆)-alkyl, heteroaryl-N(R³)-(C₁-C₆)-alkyl, heterocyclyl-N(R³)-(C₁-C₆)-alkyl, phenyl-S(O)ₙ-(C₁-C₆)-alkyl, heteroaryl-S(O)ₙ-(C₁-C₆)-alkyl, heterocyclyl-S(O)ₙ-(C₁-C₆)-alkyl, where the fifteen latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O-(C₁-C₆)-alkyl, and where heterocyclyl bears n oxo groups,
R³ is hydrogen, (C₁-C₆)-alkyl, halo-(C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl or phenyl,
R⁴ is (C₁-C₆)-alkyl, halo-(C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl or phenyl,
R⁵ is hydrogen or (C₁-C₄)-alkyl,
R⁶ is (C₁-C₄)-alkyl,
R⁷ is acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₃-C₆)-cycloalkyl, or heteroaryl or heterocyclyl each substituted by s radicals from the group consisting of methyl, ethyl, methoxy, trifluoromethyl and halogen,
n is 0, 1 or 2,
m is 0, 1, 2, 3 or 4,
s is 0, 1, 2 or 3.

2. Sulfinylaminobenzamides according to Claim 1, in which
Q is a Q1, Q2, Q3 or Q4 radical, 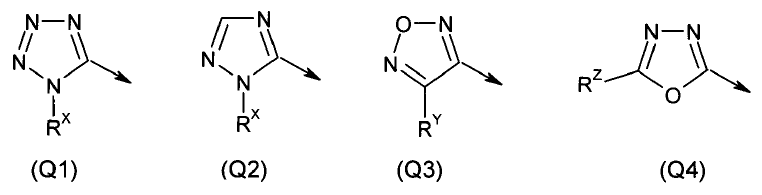
X is nitro, halogen, cyano, (C₁-C₆)-alkyl, halo-(C₁-C₆)-alkyl, (C₂-C₆)-alkenyl, (C₂-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, NC-(C₁-C₆)-alkyl, R¹O-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six latter radicals are each substituted by s radicals from the group consisting of nitro, halogen, cyano, thiocyanato, (C₁-C₆)-alkyl, halo-(C₁-C₆)-alkyl, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
Z is hydrogen, nitro, halogen, cyano, (C₁-C₆)-alkyl, halo-(C₁-C₆)-alkyl, (C₂-C₆)-alkenyl, (C₂-C₆) -alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, NC-(C₁-C₆)-alkyl, R¹O-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six latter radicals are each substituted by s radicals from the group consisting of nitro, halogen, cyano, thiocyanato, (C₁-C₆)-alkyl, halo-(C₁-C₆)-alkyl, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
W is hydrogen, halogen, nitro, cyano, (C₁-C₆)-alkyl, halo-(C₁-C₆)-alkyl, (C₃-C₇)-cycloalkyl, (C₁-C₆)-alkoxy, (C₁-C₆)-alkyl-(O)ₙS-, R¹O(O)C, (R¹)₂N, R¹(O)C(R¹)N or R²(O)₂S(R¹)N,
R and R' are each independently (C₁-C₆)-alkyl, halo-(C₁-C₆)-alkyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, (C₁-C₆)-alkoxy-(C₁-C₆)-alkyl, halo-(C₁-C₆)-alkoxy-(C₁-C₆)-alkyl, phenyl, heteroaryl or heterocyclyl, where the three latter radicals are each substituted by s radicals from the group consisting of nitro, halogen, (C₁-C₆)-alkyl, halo-(C₁-C₆)-alkyl, R¹O(O)C, (R¹)₂N(O)C, R¹O, (R¹)₂N, R²(O)ₙS and R¹O-(C₁-C₆)-alkyl, and where heterocyclyl bears n oxo groups,
or R and R' together with the sulfur atom to which they are bonded form a 3- to 8-membered unsaturated, semisaturated or saturated ring which contains, apart from the carbon atoms and apart from the sulfur atom of the sulfoximino group, in each case m ring members from the group consisting of N(R¹), 0 and S(O)ₙ, and where this ring in each case is substituted by s radicals from the group consisting of halogen, (C₁-C₆)-alkyl, halo-(C₁-C₆)-alkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where this ring bears n oxo groups,
R'' is hydrogen,
R^{X} is (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, where the six aforementioned radicals are each substituted by s radicals from the group consisting of R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyl, heteroaryl, heterocyclyl and phenyl, where the four latter radicals themselves are in turn substituted by s radicals from the group consisting of (C₁-C₆)-alkyl, halo-(C₁-C₆)-alkyl, (C₁-C₆)-alkoxy and halogen, and where heterocyclyl bears n oxo groups,
or R^{X} is (C₃-C₇)-cycloalkyl, where this radical in each case is substituted by s radicals from the group consisting of halogen, (C₁-C₆)-alkyl and halo-(C₁-C₆)-alkyl,
R^{Y} is hydrogen, (C₁-C₆)-alkyl, halo-(C₁-C₆)-alkyl, (C₃-C₇)-cycloalkyl, (C₁-C₆)-alkoxy, methoxycarbonyl, methoxycarbonylmethyl, halogen, amino, aminocarbonyl or methoxymethyl,
R^{Z} is hydrogen, (C₁-C₆)-alkyl, R¹O-(C₁-C₆)-alkyl, R⁷CH₂, (C₃-C₇)-cycloalkyl, halo-(C₁-C₆)-alkyl, R¹O, R¹(H)N, methoxycarbonyl, acetylamino or methylsulfonyl,
R¹ is hydrogen, (C₁-C₆)-alkyl, halo-(C₁-C₆)-alkyl, (C₃-C₆)-cycloalkyl, halo-(C₃-C₆)-cycloalkyl, (C₃-C₆)-cycloalkyl-(C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine latter radicals are each substituted by s radicals from the group consisting of nitro, halogen, (C₁-C₆)-alkyl, halo-(C₁-C₆)-alkyl, R³O(O)C, (R³)₂N(O)C, R³O, (R³)₂N, R⁴(O)ₙS and R³O-(C₁-C₆)-alkyl, and where heterocyclyl bears n oxo groups,
R² is (C₁-C₆)-alkyl, halo-(C₁-C₆)-alkyl, (C₃-C₆)-cycloalkyl, halo-(C₃-C₆)-cycloalkyl, (C₃-C₆)-cycloalkyl-(C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine latter radicals are each substituted by s radicals from the group consisting of nitro, halogen, (C₁-C₆)-alkyl, halo-(C₁-C₆)-alkyl, R³O(O)C, (R³)₂N(O)C, R³O, (R³)₂N, R⁴(O)ₙS and R³O-(C₁-C₆)-alkyl, and where heterocyclyl bears n oxo groups,
R³ is hydrogen or (C₁-C₆)-alkyl,
R⁴ is (C₁-C₆)-alkyl,
R⁵ is hydrogen or (C₁-C₄)-alkyl,
R⁷ is acetoxy, acetamido, methoxycarbonyl or (C₃-C₆)-cycloalkyl,
n is 0, 1 or 2,
m is 0, 1 or 2,
s is 0, 1, 2 or 3.

3. Sulfinylaminobenzamides according to Claim 1 or 2, in which
Q is a Q1, Q2, Q3 or Q4 radical, 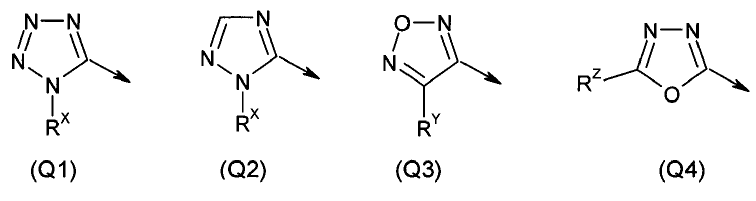
X is nitro, halogen, methyl, ethyl, n-propyl, isopropyl, trifluoromethyl, difluoromethyl, chlorodifluoromethyl, dichlorofluoromethyl, trichloromethyl, pentafluoroethyl, heptafluoroisopropyl, cyclopropyl, hydroxycarbonyl, methoxycarbonyl, ethoxycarbonyl, methoxy, ethoxy, methylsulfanyl, methylsulfinyl, methylsulfonyl, methoxymethyl, ethoxymethyl, methoxyethyl, methoxyethoxymethyl, methylthiomethyl, methylsulfinylmethyl or methylsulfonylmethyl,
Z is hydrogen, nitro, cyano, halogen, methyl, ethyl, n-propyl, isopropyl, trifluoromethyl, difluoromethyl, chlorodifluoromethyl, dichlorofluoromethyl, trichloromethyl, pentafluoroethyl, heptafluoroisopropyl, cyclopropyl, hydroxycarbonyl, methoxycarbonyl, ethoxycarbonyl, methoxy, ethoxy, methylsulfanyl, methylsulfinyl or methylsulfonyl,
W is hydrogen, chlorine or methyl,
R and R' are each independently methyl, ethyl or n-propyl,
or
R and R' together with the sulfur atom to which they are bonded form a 5- or 6-membered ring which, apart from the carbon atoms and apart from the sulfur atom of the sulfoximino group, contains m oxygen atoms,
R'' is hydrogen,
R^{X} is methyl, ethyl, n-propyl, prop-2-en-1-yl, methoxyethyl, ethoxyethyl or methoxyethoxyethyl,
R^{Y} is methyl, ethyl, n-propyl, chlorine or amino,
R^{Z} is methyl, ethyl, n-propyl or methoxymethyl,
m is 0 or 1.

4. Herbicidal compositions, **characterized by** a herbicidally active content of at least one compound of the formula (I) according to any of Claims 1 to 3.

5. Herbicidal compositions according to Claim 4 in a mixture with formulation auxiliaries.

6. Herbicidal compositions according to Claim 4 or 5, comprising at least one further pesticidally active substance from the group of insecticides, acaricides, herbicides, fungicides, safeners and growth regulators.

7. Herbicidal compositions according to Claim 6, comprising a safener.

8. Herbicidal compositions according to Claim 7, comprising cyprosulfamide, cloquintocet-mexyl, mefenpyr-diethyl or isoxadifen-ethyl.

9. Herbicidal compositions according to any of Claims 6 to 8, comprising a further herbicide.

10. Method of controlling unwanted plants, **characterized in that** an effective amount of at least one compound of the formula (I) according to any of Claims 1 to 3 or of a herbicidal composition according to any of Claims 4 to 9 is applied to the plants or to the site of the unwanted vegetation.

11. Use of compounds of the formula (I) according to any of Claims 1 to 3 or of herbicidal compositions according to any of Claims 4 to 9 for controlling unwanted plants.

12. Use according to Claim 11, **characterized in that** the compounds of the formula (I) are used for controlling unwanted plants in crops of useful plants.

13. Use according to Claim 12, **characterized in that** the useful plants are transgenic useful plants.

## Revendications

1. Sulfinylaminobenzamides de formule (I) ou leurs sels 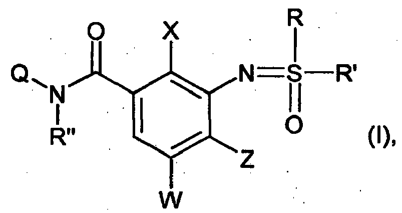 où les symboles et les indices présentent les significations suivantes :
Q signifie un radical Q1, Q2, Q3 ou Q4, 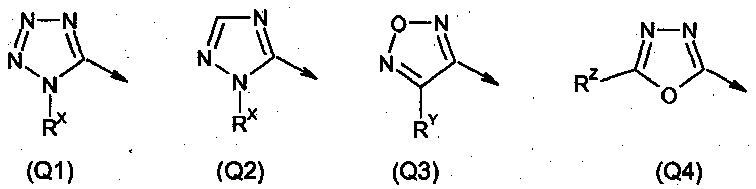
X signifie nitro, halogéno, cyano, thiocyano, (C₁-C₆)-alkyle, halogéno-(C₁-C₆)-alkyle, (C₂-C₆)-alcényle, halogéno-(C₂-C₆)-alcényle, (C₂-C₆)-alcynylé, halogéno-(C₃-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹) N (0) C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂NOC(R¹)N(O)₂S, (R⁵O)₂(O)P, R¹(O)C-(C₁-C₆)-alkyle, R¹O(O)C-(C₁-C₆)-alkyle, (R¹)₂N(O)C-(C₁-C₆)-alkyle, (R¹O)(R¹)N(O)C-(C₁-C₆)-alkyle, (R¹)₂N(R¹)N(O)C-(C₁-C₆)-alkyle, R¹(O)C(R¹)N(O)C-(C₁-C₆)-alkyle, R²O(O)C(R¹)N(O)C-(C₁-C₆)-alkyle, (R¹)₂N(O)C(R¹)N(O)C-(C₁-C₆)-alkyle, R²(O)₂S(R¹)N(O)C-(C₁-C₆)-alkyle, R¹O(O)₂S(R¹)N(O)C-(C₁-C₆)-alkyle, (R¹)₂N(O)₂S(R¹)N(O)C-(C₁-C₆)-alkyle, NC-(C₁-C₆)-alkyle, R¹O-(C₁-C₆)-alkyle, R¹(O)CO-(C₁-C₆)-alkyle, R²(O)₂SO-(C₁-C₆)-alkyle, R²O(O)CO-(C₁-C₆)-alkyle, (R¹)₂N(O)CO-(C₁-C₆)-alkyle, (R¹)₂N-(C₁-C₆)-alkyle, R¹(O)C(R¹)N-(C₁-C₆)-alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆)-alkyle, R¹O(O)₂S(R¹)N-(C₁-C₆)-alkyle, (R¹)₂N(O)₂S(R¹)N-(C₁-C₆)-alkyle, R₂(O)ₙS-(C₁-C₆)-alkyle, R¹O(O)₂S-(C₁-C₆)-alkyle, (R¹)₂N(O)₂S-(C₁-C₆)-alkyle, R¹(O)C(R¹)N(O)₂S-(C₁-C₆)-alkyle, R²O(O)C(R¹)N(O)₂S-(C₁-C₆)-alkyle, (R¹)₂N(O)C(R¹)N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, (C₃-C₆)-cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle ; et hétérocyclyle portant n groupes oxo,
Z signifie hydrogène, nitro, halogéno, cyano, thiocyano, (C₁-C₆)-alkyle, halogéno-(C₁-C₆)-alkyle, (C₂-C₆)-alcényle, halogéno- (C₂-C₆)-alcényle, (C₂-C₆)-alcynyle, halogéno-(C₃-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)n, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-alkyle, R¹O(O)C-(C₁-C₆)-alkyle, (R¹)₂N(O)C-(C₁-C₆)-alkyle, (R¹O)(R¹)N(O)C-(C₁-C₆)-alkyle, (R¹)₂N(R¹)N(O)C-(C₁-C₆)-alkyle, R¹(O)C(R¹)N(O)C-(C₁-C₆)-alkyle, R²O(O)C(R¹)N(O)C-(C₁-C₆)-alkyle, (R¹)₂N(O)C(R¹)N(O)C-(C₁-C₆)-alkyle, R²(O)₂S(R¹)N(O)C-(C₁-C₆)-alkyle, R¹O(O)₂S(R¹)N(O)C-(C₁-C₆)-alkyle, (R¹)₂N(O)₂S(R¹)N(O)C-(C₁-C₆)-alkyle, NC-(C₁-C₆)-alkyle, R¹O-(C₁-C₆)-alkyle, R¹(O)CO-(C₁-C₆)-alkyle, R²(O)₂SO-(C₁-C₆)-alkyle, R²O(O)CO-(C₁-C₆)-alkyle, (R¹)₂N(O)CO-(C₁-C₆)-alkyle, (R¹)₂N-(C₁-C₆)-alkyle, R¹(O)C(R¹)N-(C₁-C₆)-alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆)-alkyle, R¹O(O)₂S(R¹)N-(C₁-C₆)-alkyle, (R¹)₂N(O)₂S(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, R¹(O)C(R¹)N(O)₂S-(C₁-C₆)-alkyle, R²O(O)C(R¹)N(O)₂S-(C₁-C₆)-alkyle, (R¹)₂N(O)C(R¹)N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno- (C₁-C₆) -alkyle, (C₃-C₆) -cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle; et hétérocyclyle portant n groupes oxo,
W signifie hydrogène, halogéno, nitro, cyano, thiocyano, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₇)-cycloalkyle, halogéno-(C₃-C₇)-cycloalkyle, (C₁-C₆)-alcoxy, halogéno-(C₁-C₆)-alcoxy, (C₁-C₆)-alkyl-(O)ₙS-, (C₁-C₆)-halogénoalkyl-(O)ₙS-, (C₁-C₆)-alcoxy-(C₁-C₄)-alkyle, (C₁-C₆)-alcoxy-(C₁-C₄)-halogénoalkyle, R¹(O)C, R¹(R¹ON=)C, R¹O(O)C, (R¹)₂N, R¹(O)C(R¹)N ou R²(O)₂S(R¹)N,
R et R' signifient, indépendamment l'un de l'autre, à chaque fois (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, (C₁-C₆)-alcoxy-(C₁-C₆)-alkyle, halogéno-(C₁-C₆)-alcoxy-(C₁-C₆)-alkyle, phényle, hétéroaryle ou hétérocyclyle, les trois derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, (C₃-C₆)-cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle ; et hétérocyclyle portant n groupes oxo, ou
R et R' forment, ensemble avec l'atome de soufre auquel ils sont liés, un cycle de 3 à 8 chaînons, insaturé, partiellement saturé ou saturé qui contient, outre les atomes de carbone et outre l'atome de soufre du groupe sulfoximino, à chaque fois m chaînons de cycle du groupe constitué par N(R¹), 0 et S(O)ₙ ; et ce cycle étant substitué à chaque fois par s radicaux du groupe constitué par nitro, halogéno, cyano, thiocyano, (C₁-C₆)-alkyle, halogéno-(C₁-C₆) -alkyle, (C₃-C₆)-cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle ; et ce cycle portant n groupes oxo,
R" signifie hydrogène, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C-(C₁-C₆)-alkyle, R¹O(O)C-(C₁-C₆)-alkyle, (R¹)₂N(O)C-(C₁-C₆)-alkyle, NC-(C₁-C₆)-alkyle, R¹O-(C₁-C₆)-alkyle, R¹(O)CO-(C₁-C₆)-alkyle, R²(O)₂SO-(C₁-C₆)-alkyle, (R¹)₂N-(C₁-C₆)-alkyle, R¹(O)C(R¹)N-(C₁-C₆)-alkyle, R²(O)₂S(R¹)N-(C₁-C₆) -alkyle, R²(O)ₙS-(C₁-C₆)-alkyle, R¹O(O)₂S-(C₁-C₆)-alkyle, (R¹)₂N(O)₂S-(C₁-C₆)-alkyle, R¹(O)C, R¹O(O)C, (R¹)₂N(O)C, R¹O, (R¹)₂N, R²O(O)C(R¹)N, (R¹)₂N(O)C(R¹)N, R²(O)₂S ; ou benzyle à chaque fois substitué par s radicaux du groupe constitué par méthyle, éthyle, méthoxy, nitro, trifluorométhyle et halogéno,
R^{x} signifie (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, les six radicaux susmentionnés étant à chaque fois substitués par s radicaux du groupe constitué par nitro, cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyle, hétéroaryle, hétérocyclyle et phényle et les quatre derniers radicaux mentionnés étant substitués par s radicaux du groupe constitué par (C₁-C₆)-alkyle, halogéno-(C₁-C₆)-alkyle, (C₁-C₆)-alcoxy, halogéno-(C₁-C₆)-alcoxy et halogéno ; et hétérocyclyle portant n groupes oxo, ou
R^{x} signifie (C₃-C₇)-cycloalkyle, hétéroaryle, hétérocyclyle ou phényle, les quatre radicaux susmentionnés étant à chaque fois substitués par s radicaux du groupe constitué par halogéno, nitro, cyano, (C₁-C₆)-alkyle, halogéno-(C₁-C₆)-alkyle, (C₃-C₆)-cycloalkyle, (C₁-C₆)-alkyl-S(O)ₙ, (C₁-C₆)-alcoxy, halogéno-(C₁-C₆)-alcoxy et (C₁-C₆)-alcoxy-(C₁-C₄)-alkyle,
R^{Y} signifie hydrogène, (C₁-C₆)-alkyle, halogéno-(C₁-C₆)-alkyle, (C₂-C₆)-alcényle, halogéne-(C₂-C₆)-alcényle, (C₂-C₆)-alcynyle, halogéno-(C₃-C₆)-alcynyle, (C₃-C₇)-cycloalkyle, (C₁-C₆)-alcoxy, halogéno-(C₁-C₆)-alcoxy, (C₂-C₆)-alcényloxy, (C₂-C₆)-alcynyloxy, cyano, nitro, méthylsulfanyle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogéno, amino, aminocarbonyle, méthylaminocarbonyle, diméthylaminocarbonyle, méthoxyméthyle ; ou hétéroaryle, hétérocyclyle ou phényle à chaque fois substitué par s radicaux du groupe constitué par (C₁-C₆)-alkyle, halogéno-(C₁-C₆)-alkyle, (C₁-C₆)-alcoxy, halogéno-(C₁-C₆)-alcoxy et halogéno ; et hétérocyclyle portant n groupes oxo,
R^{z} signifie hydrogène, (C₁-C₆)-alkyle, R¹O-(C₁-C₆)-alkyle, R⁷CH₂, (C₃-C₇) -cycloalkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, R¹O, R¹(H)N, méthoxycarbonyle, éthoxycarbonyle, méthylcarbonyle, diméthylamino, trifluorométhylcarbonyle, acétylamino, méthylsulfanyle, méthylsulfinyle, méthylsulfonyle ; ou hétéroaryle, hétérocyclyle, benzyle ou phényle à chaque fois substitué par s radicaux du groupe constitué par halogéno, nitro, cyano, (C₁-C₆)-alkyle, halogéno-(C₁-C₆)-alkyle, (C₃-C₆)-cycloalkyle, (C₁-C₆)-alkyl-S(O)ₙ, (C₁-C₆)-alcoxy, halogéno-(C₁-C₆)-alcoxy et (C₁-C₆)-alcoxy-(C₁-C₄)-alkyle ; hétérocyclyle portant n groupes oxo,
R¹ signifie hydrogène, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, (C₃-C₆)-cycloalcényle, halogéno-(C₃-C₆)-cycloalkyle, (C₃-C₆)-cycloalkyl-(C₁-C₆)-alkyle, (C₁-C₆)-alkyl-O-(C₁-C₆)-alkyle, cycloalkyl-(C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, phényl-N(R³)-(C₁-C₆)-alkyle, hétéroaryl-N(R³)-(C₁-C₆)-alkyle, hétérocyclyl-N(R³)-(C₁-C₆)-alkyle, phényl-S(O)ₙ-(C₁-C₆)-alkyle, hétéroaryl-S(O)ₙ-(C₁-C₆)-alkyle, hétérocyclyl-S(O)ₙ-(C₁-C₆)-alkyle, les quinze derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, (C₃-C₆)-cycloalkyle, R³O(O)C, (R³)₂N(O)C, R³O, (R³)₂N, R⁴(O)ₙS, R³O(O)₂S, (R³)₂N(O)₂S et R³O-(C₁-C₆)-alkyle ; et hétérocyclyle portant n groupes oxo,
R² signifie (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, (C₃-C₆)-cycloalcényle, halogéno-(C₃-C₆)-cycloalkyle, (C₃-C₆)-cycloalkyl-(C₁-C₆)-alkyle, (C₁-C₆)-alkyl-O-(C₁-C₆)-alkyle, cycloalkyl-(C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, phényl-N(R³)-(C₁**-**C₆)-alkyle, hétéroaryl-N(R³)-(C₁-C₆)-alkyle, hétérocyclyl-N(R³)-(C₁-C₆)-alkyle, phényl-S(O)ₙ-(C₁-C₆)-alkyle, hétéroaryl-S(O)ₙ-(C₁-C₆)-alkyle, hétérocyclyl-S(O)ₙ-(C₁-C₆)-alkyle, les quinze derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, (C₃-C₆) -cycloalkyle, R³O(O)C, (R³)₂N(O)C, R³O, (R³)₂N, R⁴(O)ₙS, R³O(O)₂S, (R³)₂N(O)₂S et R³O-(C₁-C₆)-alkyle ; et hétérocyclyle portant n groupes oxo,
R³ signifie hydrogène, (C₁-C₆)-alkyle, halogéno-(C₁-C₆)-alkyle, (C₂-C₆)-alcényle, (C₂-C₆)-alcynyle, (C₃-C₆)-cycloalkyle, (C₃-C₆)-cycloalkyl-(C₁-C₆)-alkyle ou phényle,
R⁴ signifie (C₁-C₆)-alkyle, halogéno-(C₁-C₆)-alkyle, (C₂-C₆)-alcényle, (C₂-C₆)-alcynyle, (C₃-C₆)-cycloalkyle, (C₃-C₆)-cycloalkyl-(C₁-C₆)-alkyle ou phényle,
R⁵ signifie hydrogène ou (C₁-C₄)-alkyle,
R⁶ signifie (C₁-C₄)-alkyle,
R⁷ signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, (C₃-C₆)-cycloalkyle ; ou hétéroaryle ou hétérocyclyle à chaque fois substitué par s radicaux du groupe constitué par méthyle, éthyle, méthoxy, trifluorométhyle et halogéno,
n vaut 0, 1 ou 2,
m vaut 0, 1, 2, 3 ou 4,
s vaut 0, 1, 2 ou 3.

2. Sulfinylaminobenzamides selon la revendication 1, dans laquelle
Q signifie un radical Q1, Q2, Q3 ou Q4, 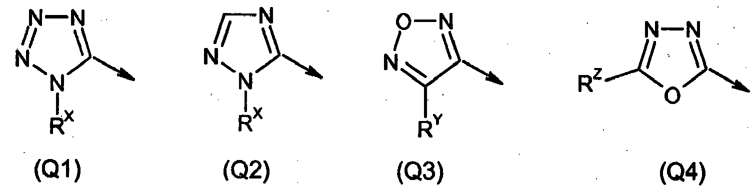
X signifie nitro, halogéno, cyano, (C₁-C₆)-alkyle, halogéno-(C₁-C₆)-alkyle, (C₂-C₆)-alcényle, (C₂-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹ (O)C-(C₁-C₆)-alkyle, R¹O(O)C-(C₁-C₆)-alkyle, (R¹)₂N(O)C-(C₁-C₆)-alkyle, NC-(C₁-C₆)-alkyle, R¹O-(C₁-C₆) -alkyle, (R¹)₂N-(C₁-C₆)-alkyle, R¹(O)C(R¹)N-(C₁-C₆)-alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, R¹O, (R¹)₂N, R²(O)ₙS, R¹O(O)₂S, (R¹)₂N(O)₂S et R¹O-(C₁-C₆)-alkyle ; et hétérocyclyle portant n groupes oxo,
Z signifie hydrogène, nitro, halogéno, cyano, (C₁-C₆)-alkyle, halogéno-(C₁-C₆)-alkyle, (C₂-C₆)-alcényle, (C₂-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-alkyle, R¹O(O)C-(C₁-C₆)-alkyle, (R¹)₂N(O)C-(C₁-C₆)-alkyle, NC-(C₁-C₆)-alkyle, R¹O-(C₁-C₆)-alkyle, (R¹)₂N-(C₁-C₆)-alkyle, R¹(O)C(R¹)N-(C₁-C₆)-alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, R¹O, (R¹)₂N, R²(O)ₙS, R¹O(O)₂S, (R¹)₂N(O)₂S et R¹O-(C₁-C₆)-alkyle ; et hétérocyclyle portant n groupes oxo,
W signifie hydrogène, halogéno, nitro, cyano, (C₁-C₆)-alkyle, halogéno-(C₁-C₆)-alkyle, (C₃-C₇)-cycloalkyle, (C₁-C₆)-alcoxy, (C₁-C₆)-alkyl-(O)ₙS-, R¹O(O)C, (R¹)₂N, R¹(O)C(R¹)N ou R²(O)₂S(R¹)N,
R et R' signifient, indépendamment l'un de l'autre, à chaque fois (C₁-C₆)-alkyle, halogéno-(C₁-C₆)-alkyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, (C₁-C₆)-alcoxy-(C₁-C₆)-alkyle, halogéno-(C₁-C₆)-alcoxy-(C₁-C₆)-alkyle, phényle, hétéroaryle ou hétérocyclyle, les trois derniers radicaux mentionnés étant à chaque fois substitués par s radicaux du groupe constitué par nitro, halogéno, (C₁-C₆)-alkyle, halogéno-(C₁-C₆)-alkyle, R¹O(O)C, (R¹)₂N(O)C, R¹O, (R¹)₂N, R²(O)ₙS et R¹O-(C₁-C₆)-alkyle ; et hétérocyclyle portant n groupes oxo, ou
R et R' forment, ensemble avec l'atome de soufre auquel ils sont liés, un cycle de 3 à 8 chaînons, insaturé, partiellement saturé ou saturé qui contient, outre les atomes de carbone et outre l'atome de soufre du groupe sulfoximino, à chaque fois m chaînons de cycle du groupe constitué par N(R¹), 0 et S(O)ₙ ; et ce cycle étant substitué à chaque fois par s radicaux du groupe constitué par halogéno, (C₁-C₆)-alkyle, halogéno-(C₁-C₆)-alkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle ; et ce cycle portant n groupes oxo,
R" signifie hydrogène,
R^{X} signifie (C₁-C₆)-alkyle, halogéno-(C₁-C₆) -alkyle, (C₂-C₆)-alcényle, halogéno-(C₂-C₆)-alcényle, (C₂-C₆)-alcynyle, halogéno-(C₃-C₆)-alcynyle, les six radicaux susmentionnés étant à chaque fois substitués par s radicaux du groupe constitué par R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyle, hétéroaryle, hétérocyclyle et phényle, les quatre derniers radicaux mentionnés étant eux-mêmes à leur tour substitués par s radicaux du groupe constitué par (C₁-C₆)-alkyle, halogéno-(C₁-C₆)-alkyle, (C₁-C₆)-alcoxy et halogéno ; et hétérocyclyle portant n groupes oxo, ou
R^{X} signifie (C₃-C₇)-cycloalkyle, ce radical étant à chaque fois substitué par s radicaux du groupe constitué par halogéno, (C₁-C₆)-alkyle et halogéno-(C₁-C₆)-alkyle,
R^{Y} signifie hydrogène, (C₁-C₆)-alkyle, halogéno-(C₁-C₆)-alkyle, (C₃-C₇)-cycloalkyle, (C₁-C₆)-alcoxy, méthoxycarbonyle, méthoxycarbonylméthyle, halogéno, amino, aminocarbonyle ou méthoxyméthyle,
R^{Z} signifie hydrogène, (C₁-C₆)-alkyle, R¹O-(C₁-C₆)-alkyle, R⁷CH₂, (C₃-C₇)-cycloalkyle, halogéno-(C₁-C₆)-alkyle, R¹O, R¹(H)N, méthoxycarbonyle, acétylamino ou méthylsulfonyle,
R¹ signifie hydrogène, (C₁-C₆)-alkyle, halogéno-(C₁-C₆)-alkyle, (C₃-C₆)-cycloalkyle, halogéno-(C₃-C₆)-cycloalkyle, (C₃-C₆)-cycloalkyl-(C₁-C₆)-alkyle, (C₁-C₆)-alkyl-O-(C₁-C₆)-alkyle, cycloalkyl-(C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, les neuf derniers radicaux mentionnés étant à chaque fois substitués par s radicaux du groupe constitué par nitro, halogéno, (C₁-C₆)-alkyle, halogéno-(C₁-C₆)-alkyle, R³O(O)C, (R³)₂N(O)C, R³O, (R³)₂N, R⁴(O)ₙS et R³O-(C₁-C₆)-alkyle ; et hétérocyclyle portant n groupes oxo,
R² signifie (C₁-C₆)-alkyle, halogéno-(C₁-C₆)-alkyle, (C₃-C₆)-cycloalkyle, halogéno-(C₃-C₆)-cycloalkyle, (C₃-C₆)-cycloalkyl-(C₁-C₆)-alkyle, (C₁-C₆)-alkyl-O-(C₁-C₆)-alkyle, cycloalkyl-(C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, les neuf derniers radicaux mentionnés étant à chaque fois substitués par s radicaux du groupe constitué par nitro, halogéno, (C₁-C₆)-alkyle, halogéno-(C₁-C₆)-alkyle, R³O(O)C, (R³)₂N(O)C, R³O, (R³)₂N, R⁴(O)ₙS et R³O-(C₁-C₆)-alkyle ; et hétérocyclyle portant n groupes oxo,
R³ signifie hydrogène ou (C₁-C₆)-alkyle,
R⁴ signifie (C₁-C₆)-alkyle,
R⁵ signifie hydrogène ou (C₁-C₄)-alkyle,
R⁷ signifie acétoxy, acétamido, méthoxycarbonyle ou (C₃-C₆)-cycloalkyle,
n vaut 0, 1 ou 2,
m vaut 0, 1 ou 2,
s vaut 0, 1, 2 ou 3.

3. Sulfinylaminobenzamides selon la revendication 1 ou 2, dans laquelle
Q signifie un radical Q1, Q2, Q3 ou Q4, 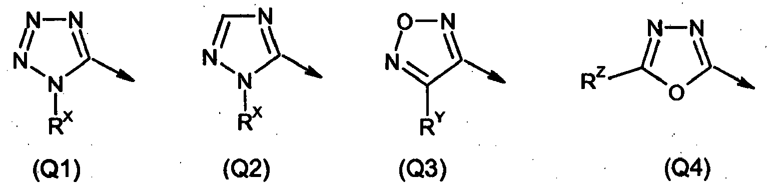
X signifie nitro, halogéno, méthyle, éthyle, n-propyle, iso-propyle, trifluorométhyle, difluorométhyle, chlorodifluorométhyle, dichlorofluorométhyle, trichlorométhyle, pentafluoroéthyle, heptafluoro-isopropyle, cyclopropyle, hydroxycarbonyle, méthoxycarbonyle, éthoxycarbonyle, méthoxy, éthoxy, méthylsulfanyle, méthylsulfinyle, méthylsulfonyle, méthoxyméthyle, éthoxyméthyle, méthoxyéthyle, méthoxyéthoxyméthyle, méthylthiométhyle, méthylsulfinylméthyle ou méthylsulfonylméthyle,
Z signifie hydrogène, nitro, cyano, halogéno, méthyle, éthyle, n-propyle, iso-propyle, trifluorométhyle, difluorométhyle, chlorodifluorométhyle, dichlorofluorométhyle, trichlorométhyle, pentafluoroéthyle, heptafluoroisopropyle, cyclopropyle, hydroxycarbonyle, méthoxycarbonyle, éthoxycarbonyle, méthoxy, éthoxy, méthylsulfanyle, méthylsulfinyle ou méthylsulfonyle,
W signifie hydrogène, chlore ou méthyle,
R et R' signifient, indépendamment l'un de l'autre, à chaque fois méthyle, éthyle ou n-propyle, ou
R et R' forment, ensemble avec l'atome de soufre auquel ils sont liés, un cycle de 5 à 6 chaînons saturé qui contient, outre les atomes de carbone et outre l'atome de soufre du groupe sulfoximino, m atomes d'oxygène,
R" signifie hydrogène,
R^{X} signifie méthyle, éthyle, n-propyle, prop-2-én-1-yle, méthoxyéthyle, éthoxyéthyle ou méthoxyéthoxyéthyle,
R^{Y} signifie méthyle, éthyle, n-propyle, chlore ou amino,
R^{Z} signifie méthyle, éthyle, n-propyle ou méthoxyméthyle,
m vaut 0 ou 1.

4. Agents herbicides, **caractérisés par** une teneur active en tant qu'herbicide en au moins un composé de formule (I) selon l'une quelconque des revendications 1 à 3.

5. Agents herbicides selon la revendication 4 en mélange avec des adjuvants de formulation.

6. Agents herbicides selon la revendication 4 ou 5 contenant au moins une autre substance active en tant que pesticide du groupe formé par les insecticides, les acaricides, les herbicides, les fongicides, les phytoprotecteurs et les régulateurs de croissance.

7. Agents herbicides selon la revendication 6 contenant un phytoprotecteur.

8. Agents herbicides selon la revendication 7, contenant du cyprosulfamide, du cloquintocet-mexyl, du méfenpyr-diéthyl ou de l'isoxadifen-éthyl.

9. Agents herbicides selon l'une quelconque des revendications 6 à 8 contenant un autre herbicide.

10. Procédé pour lutter contre des plantes non souhaitées, **caractérisé en ce qu'**on applique une quantité active d'au moins un composé de formule (I) selon l'une quelconque des revendications 1 à 3 ou d'un agent herbicide selon l'une quelconque des revendications 4 à 9 sur les plantes ou sur le lieu de la croissance non souhaitée des plantes.

11. Utilisation de composés de formule (I) selon l'une quelconque des revendications 1 à 3 ou d'agents herbicides selon l'une quelconque des revendications 4 à 9 pour lutter contre des plantes non souhaitées.

12. Utilisation selon la revendication 11, **caractérisée en ce que** les composés de formule (I) sont utilisés pour lutter contre des plantes non souhaitées dans les cultures de plantes utiles.

13. Utilisation selon la revendication 12, **caractérisée en ce que** les plantes utiles sont des plantes utiles transgéniques.
